# EUROPÄISCHE PATENTSCHRIFT

(11) **EP 2 709 558 B1**
(45) Veröffentlichungstag und Bekanntmachung des Hinweises auf die Patenterteilung: **05.01.2022**
(21) Anmeldenummer: 12729848.7
(22) Anmeldetag: 15.05.2012
(51) Int. Cl.: A61F 2/16

(54) **INJEKTOR ZUM IMPLANTIEREN EINER INTRAOKULARLINSE**
INJECTOR FOR IMPLANTING AN INTRAOCULAR LENS
INJECTEUR POUR L'IMPLANTATION D'UNE LENTILLE INTRAOCULAIRE

(30) Priorität: 18.05.2011 DE 102011101940
(43) Veröffentlichungstag der Anmeldung: 26.03.2014
(73) Patentinhaber: Iolution GmbH, 22761 Hamburg (DE)
(72) Erfinder: MAROSCHECK, Christoph, 22769 Hamburg (DE); BINDER, Helmut, 12203 Berlin (DE)
(74) Vertreter: Friderichs, Gunther
(86) Internationale Anmeldenummer: PCT/DE2012/000501
(87) Internationale Veröffentlichungsnummer: WO 2012/155887

(56) Entgegenhaltungen:
- WO-A1-00/45746
- US-A1- 2003 212 406
- US-A1- 2004 243 141
- US-A1- 2009 043 313
- US-A1- 2009 204 122
- US-A1- 2009 292 294

## Beschreibung

### Beschreibung der Erfindung

Die vorliegende Erfindung betrifft ein Injektorsystem zum Implantieren einer Intraokularlinse in ein Auge und ein Magazin für das Injektorsystem.

### Hintergrund der Erfindung

Intraokularlinsenstellen Linsenimplantate oder künstliche Linsen zum Ersatz der natürlichen Linsen eines menschlichen Auges dar. Sie werden insbesondere als Ersatz für die unter einer Augentrübung (Katarakt) erkrankten Linsen des Auges eingesetzt. Mittels einer Operation werden die erkrankten Linsen entfernt und die Intraokularlinsen eingesetzt. Diese werden zum Beispiel mittels eines sogenannten Injektors in das Auge eingesetzt bzw. eingeführt. Hierbei ist es von Bedeutung, - dass der Operationsschnitt, durch welchen eine Intraokularlinse implantiert wird, möglichst klein ist (zum Beispiel etwa 3 mm). Dadurch kann ein möglichst schneller und komplikationsfreier Heilungsprozess gewährleisten sein und gegebenenfalls ist auch keine Naht erforderlich.

Um Intraokularlinsen, die im Allgemeinen einen Durchmesser von ca. 5 bis 6 mm besitzen, implantieren zu können, müssen diese faltbar sein, damit sie durch den kleinen Schnitt von etwa 3 mm hindurchpassen. Ein Injektor zum Falten und

Einbringen einer gefalteten Linse in das menschliche Auge ist zum Beispiel in der internationalen Patentanmeldung WO' 00/45746 A1 beschrieben.

Dort ist ein Injektor zum Implantieren bzw. Einbringen einer zeitweilig gefalteten Intraokularlinse beschrieben, mit welchem die gefaltete Linse durch eine Schnittöffnung im Auge mit der erforderlichen Größe von etwa 3 mm in die Linsenkapsel des Auges einsetzbar ist. Der Injektor umfasst einen Körper, der aus einem dickeren

Einbring- und Halteteil und einem einführseitigen, dünneren Einführrohr besteht und eine axiale Durchgangsöffnung als Transportkanal besitzt. Zudem umfasst der Injektor einen Schieber, welcher in dem Transportkanal axial verschiebbar angeordnet ist. Weiterhin umfasst der Injektor eine in dem Einbring- und Halteteil quer zur Achse des Transportkanals vorgesehene, radiale Einbringöffnung als Einbringkanal für die Linse. Die Einbringöffnung steht in Verbindung mit der Transport-Durchgangsöffnung. In der Einbringöffnung liegt die ungefaltete Linse für den Transport flach auf einer

Auflagefläche auf und wird über eine längsmittig zur Linse sich erstreckende Halterippe festgehalten. Die Halterippe ist eine sich plattenförmig radial erstreckende Faltrippe, die durch die Einbringöffnung bis in den Transportkanal radial eindrückbar ist, so dass die Linse um die Halterippe gefaltet vollständig in den Transportkanal eingebracht wird .

Der dort beschriebene Injektor gewährleistet ein sicheres Implantieren einer Intraokularlinse in ein menschliches und/oder tierisches Auge. Das Laden des Injektors bzw. das Einlegen der Intraokularlinse in den Injektor kann erst kurz vor der Operation durch den operierenden Augenarzt oder eine unterstützende OP-Schwester erfolgen. Um die Intraokularlinse präzise in das Auge einbringen zu können, ist es aber erforderlich, dass die Linse möglichst präzise auf der Auflagefläche positioniert ist, so dass die Linse durch die Faltrippe präzise in den Transportkanal einbringbar ist.

Wird die Linse nicht ausreichend präzise geladen, kann dies zu einer unerwünschten Rotation der Linse beim Falten und Einbringen der Linse in den Transportkanal mittels der Faltrippe führen. Unter Umständen kann hierbei sogar die Linse derart geklemmt werden zwischen der Faltrippe und dem Transportkanal, dass diese unbrauchbar wird.

Daher stellt das Laden des Injektors durch den Augenarzt oder die OP-Schwester ein potentielles Risiko dar. Zudem kann die bis dahin in einer Verpackung steril gelagerte Linse beim Entnehmen aus der Verpackung und beim Einlegen in den Injektor verunreinigt werden.

Injektoren sind auch aus den Dokumenten US 2003212406 A1, US 2009292294 A1 US 2009204122 A1, WO 0045746 A1, US 2004243141 A1 und US 2009043313 A1 bekannt.

### Allgemeine Beschreibung der Erfindung

Vor dem vorstehend geschilderten Hintergrund liegt der vorliegenden Erfindung die Aufgabe zugrunde, einen Injektor für Intraokularlinsen bereitzustellen, der die vorstehend genannten Nachteile zumindest reduziert oder sogar vollständig vermeidet.

Es soll auch möglich sein, insbesondere auch bei einem nicht optimalen Laden der Linse, ein reproduzierbares Einbringen und Falten der Linse in den Transportkanal und ein reproduzierbares Einbringen und Entfalten der Linse im Auge zu gewährleisten. Gelöst werden diese Aufgaben bereits durch das Injektorsystem gemäß den den Merkmalen des Patentanspruchs 1. Vorteilhafte Ausführungsformen sind Gegenstand der Unteransprüche.

Allgemein sieht die Erfindung vor, den im Stand der Technik beschriebenen Injektor derart weiterzuentwickeln, dass die Arretierung der Intraokularlinse im Injektor auf der einen Seite und das Falten und Einbringen der Intraokularlinse in den Injektor auf der anderen Seite nicht durch dasselbe Bauteil erfolgen.

Im Detail sieht die vorliegende Erfindung einen Injektor oder ein Injektorsystem zum Einbringen oder Implantieren einer Linse in ein Auge vor, welches folgende Bestandteile umfasst:
einen Injektorkörper mit einer Vorderseite und einer Rückseite,
eine an der Vorderseite des Injektorkörpers angeordnete Kanüle, die einen Transportkanal für eine zu implantierende Linse bereitstellt, wobei dem Transportkanal eine Linse über eine vorzugsweise seitliche Eingangsöffnung zuführbar ist, ein Magazin mit einem Aufnahmebereich für wenigstens eine Linse, welche in dem Aufnahmebereich mittels einer Halterung festsetzbar ist, wobei das Magazin so angeordnet ist, dass dem Transportkanal eine Linse über die vorzugsweise seitliche Eingangsöffnung zuführbar ist, einen in das Magazin und die Eingangsöffnung einfügbaren Faltkörper zum Einschieben der Linse in den Transportkanal derart, dass die Linse zumindest abschnittsweise umden Faltkörper faltbar ist, und einen Schieber, der im Injektorkörper, vorzugsweise axial, verschiebbar angeordnet ist und über die Vorderseite des Injektorkörpers so in den Transportkanal einschiebbar ist, dass die Linse aus dem Transportkanal ausgeschoben werden kann oder ausschiebbar ist. Die Kanüle ist ein Röhrchen -oder umfasst ein Röhrchen, das zumindest abschnittsweise in ein Auge eingeführt wird und über das die Linse in das Auge eingeführt wird. Die Linse wird dazu von ihrer Ausgangsposition in dem Magazin in die Kanüle bzw. in den Transportkanal der Kanüle und somit in die Transportposition überführt. Aus der Transportposition wird die Linse mittels des Schiebers aus dem Transportkanal über die Austrittsöffnung in das Auge eingeschoben. Der Schieber zum Ausschieben der Linse aus dem Injektor kann auch als Linsenschieber bezeichnet sein. Der Transportkanal beschreibt somit wenigstens den Abschnitt im Injektor, durch den die Linse mittels des Schiebers bewegt wird. Die Kanüle kann mit dem Injektorkörper unmittelbar oder mittelbar verbunden sein.

Die Eingangsöffnung oder Einführöffnung ist vorzugsweise seitlich am Transportkanal angeordnet, zum Beispiel an der Oberseite oder der Unterseite des Transportkanals.

Die Linse wird durch den Faltkörper aus dem Magazin in den Transportkanal überführt, insbesondere durch Schieben bzw. Drücken'. Der Faltkörper ist eine Einrichtung, zum Falten der Linse und/oder zum Einschieben der Linse in den Transportkanal.

Vorzugsweise ist der Faltkörper eine Faltplatte, die auch als Faltschwert bezeichnet werden kann. Die Faltplatte oder der Faltkörper ist eine Platte bzw. ein Körper, die bzw. der so ausgeführt ist und/oder so in die Linse eingreift, dass die Linse beim Eingriff durch die Faltplatte bzw. den Faltköper um die Faltplatte bzw. den Faltkörper gefaltet wird. Die Faltplatte und der Faltkörper wird, insbesondere sofern sie bzw. er an einer Klappe angeordnet ist, auch als Faltrippe bezeichnet. Der Faltkörper oder die Faltplatte oder die Faltrippe ist vorzugsweise radial in den Transportkanal eindrückbar.

Der Faltkörper oder die Faltplatte kann als separates Bauteil bereitgestellt sein oder mit. dem Injektorsystem, vorzugsweise der Kanüle, verbunden sein.

In einer Ausführungsform ist der Faltkörper oder die Faltplatte an einer Unterseite einer Klappe angeordnet ist, welche vorzugsweise schwenkbar an der Kanüle befestigt ist, so dass eine Linse in einem heruntergeklappten Zustand der Klappe in den Transportkanal eingeschoben und zumindest abschnittsweise um den Faltkörper, oder die Faltplatte gefaltet ist. In dieser Ausführungsform, wird die Klappe nachfolgend auch kurz als Faltklappe, da sie die Faltplatte bzw. den Faltkörper trägt, bezeichnet.

In einer Ausführungsform sind an dem Faltkörper bzw. der Faltplatte und/oder im oberen Bereich des Transportkanals wenigstens zwei Rückhaltekanten für eine Linse angeordnet, welche eine Anlagefläche zumindest für einen Abschnitt der Linsenkante bzw. des Linsenrands beim Einschieben einer Linse in den Transportkanal definieren. Im Allgemeinen besitzt eine Intraokularlinse zwei Haptiken. Um ein zuverlässiges und sicheres Einbringen der Linse in das Auge zu unterstützen, sollten die Haptiken nicht von der Linse abstehen. Die Haptiken sollten sich vielmehr in einer definierten Position befinden. In einer Ausgestaltung der Erfindung sollen die Haptiken an der Linse anliegen. Sie können zum Beispiel in die Linse eingerollt sein. In Abhängigkeit von der Ausrichtung der Linse im Injektor liegt zum Beispiel eine Haptik im vorderen Bereich (die vordere Haptik) und eine Haptik im hinteren Bereich (die hintere Haptik) des Injektors.

In einer Ausführungsform der Erfindung weist der Faltkörper einen Aufnahmebereich für eine vorzugsweise hintere Haptik der Linse auf, in welchen die vorzugsweise hintere Haptik der Linse einführbar ist. Insbesondere ist bzw. wird der Aufnahmebereich für die vorzugsweise hintere Haptik der Linse durch eine Aussparung an einer Seite, vorzugsweise der Rückseite, des Faltkörpers bereitgestellt.

Um die hintere Haptik in den Aufnahmebereich zu überführen, weist das Injektorsystem in einer Ausgestaltung einen Haptikschieber auf. In einem ersten Schritt kann die hintere Haptik der Linse durch den Haptikschieber in den Aufnahmebereich in dem Faltkörper eingebracht werden. In einem zweiten Schritt wird dann die Linse durch den Schieber für die Linse aus dem Injektor ausgeschoben. Beim Ausschieben wird die gefaltete Linse beispielsweise gerollt, wobei die hintere Haptik zumindest abschnittsweise mit eingerollt wird. Die hintere Haptik befindet sich dann zumindest abschnittsweise im Inneren der zusammengerollten Linse.

Der Haptikschieber kann aber auch mit dem Schieber für die Linse gekoppelt oder koppelbar sein, so dass die hintere Haptik der Linse durch den Haptikschieber mittels einer Bewegung des Schiebers für die Linse in den Aufnahmebereich des Faltkörpers einschiebbar ist. Dadurch kann über eine, vorzugsweise einzelne, Betätigung des Schiebers die hintere Haptik definiert positioniert oder eingerollt und die Linse aus dem Injektor ausgeschoben werden.In einer Variante der Erfindung umfasst der Haptikschieber wenigstens einen Biegearm, an welchem der Schieber anliegt oder beim Ausschieben der Linse zur Anlage kommt, so dass der wenigstens eine Biegearm gegen eine Innenseite des Injektorkörpers gedrückt wird und der Haptikschieber durch den Schieber mit in Richtung der Vorderseite des Injektors bewegbar ist. Vorzugsweise besitzt der Haptikschieber zwei Biege' arme. Eine Biegearm ist beispielsweise ein Arm, der im Wesentlichen elastisch biegbar ist.

Insbesondere weist der wenigstens eine Biegearm wenigstens einen Vorsprung auf, über welchen der Biegearm gegen die Innenseite des Injektorkörpers gedrückt wird. Beim Schieben des Linsenschiebers und somit auch des Haptikschiebers in Richtung der Vorderseite des Injektors kommt der wenigstens eine Biegearm nach einer bestimmten Verschiebungsstrecke in einer Aussparung, vorzugsweise in einem Loch, der Innenseite des Injektorkörpers zum Eingriff. Damit wird der Haptikschieber von dem Schieber für die Linse entkoppelt.

Der Haptikschieber besitzt in einer weiterem Ausgestaltung an seiner Vorderseite einen Fänger für die hintere Haptik der Linse. Um eine definierte Position der vorderen Haptik in Bezug zur Linse zu erzielen, ist der Injektor insbesondere auch dadurch gekennzeichnet, dass der Faltkörper einen Anschlag für eine vordere Haptik der Linse aufweist, so dass die vordere Haptik beim Ausschieben der Linse durch den Schieber mittels des Anschlags auf der Linse zur Auflage kommt. Die vordere Haptik kann beim Auschieben der Linse ebenso in diese eingerollt werden oder einfach nur zur Auflage auf der Außenseite der Linse kommen. Insbesondere wird der Anschlag für die vordere Haptik der Linse als ein, vorzugsweise flexiber, Vorsprung an einer Unterseite des Faltkörpers bereitgestellt.

Gemäß der Erfindung ist der Faltkörper zweiteilig aufgebaut. Es wird ein Sockel bereitgestellt, in welchem das Faltelement, insbesondere mit Haptikaufnahmebereich, eingesteckt ist. Die Rückhaltekanten werden von dem Sockel bereitgestellt. Das Faltelement ist im Sockel verschiebbar angeordnet. Dies kann zum Beispiel dadurch erzielt werden, dass die Oberseite des Faltelements im Inneren des Sockels nicht an diesem anliegt. Es wird ein Zwischenraum erzeugt. In dem Zwischenraum kann zusätzlich ein flexiber oder elastischer Körper, zum Beispiel ein Schaumstoff, eingebracht sein.

Die Halterung ist eine Einrichtung zum Festsetzen der Linse bzw. zum Arretieren der Linse in einer Position im Magazin bzw. im Aufnahmebereich des Magazins, der Ausgangsposition. Die Halterung kann als ein separates Bauteil bereitgestellt oder mit dem Injektor, vorzugsweise dem Magazin, verbunden sein. In einer Variante der Erfindung ist die Halterung als eine Klappe ausgeführt, welche schwenkbar an dem Magazin angeordnet ist, wobei in einer geschlossenen Stellung der Klappe eine Linse festgesetzt ist. In der Ausführungsform der Halterung als Klappe wird die Halterung nachfolgend auch als Halteklappe bezeichnet.

In einer Ausführungsform weist die Halterung an der Seite, welche der Linse zugeordnet ist, eine Rückhalteeinrichtung und/oder eine Verdrehsicherung für eine im Aufnahmebereich zu lagernde Linse auf. Die Rückhalteeinrichtung stellt eine Sicherheitsvorrichtung dar zum Festhalten oder Arretieren der Linse in dem Magazinbzw. in dem Aufnahmebereich des Magazins, insbesondere um ein Verrutschen der Linse im Magazin oder ein Herausfallen der Linse aus dem Magazin zu vermeiden. Die genannte Verdrehsicherung soll ein Verdrehen der Linse im Magazin bzw. im Aufnahmebereich des Magazins vermeiden.

In einer Ausführungsform ist die Rückhalteeinrichtung als ein, vorzugsweise plattenförmiger, Vorsprung ausgeführt, welcher sich zumindest abschnittsweise über den Umfang einerim Aufnahmebereich festzusetzenden Linse erstreckt und eine zu dieser im Wesentlichen korrespondierende Krümmung aufweist. Vorzugsweise kommt die Rückhalteeinrichtung dabei nicht in Auflage auf den optisch relevanten Bereich der Linse. Sie kommt zumindest abschnittsweise in Auflage auf den Bereich, an dem die Haptik mit der Linse verbunden ist oder in die Linse übergeht.

In einer weiteren Ausführungsform ist die Verdrehsicherung ' als wenigstens ein Stift ausgeführt, welcher zwischen eine " Linse und einer Haptik einer Linse eingreift und vorzugsweise an der Rückhalteeinrichtung angeordnet ist.

In einer Ausgestaltung der Erfindung weist die Halterung eine Öffnung auf. Die Faltplatte ist über die Öffnung in der Halterung in das Magazin und in die Eingangsöffnung im Transportkanal einfügbar.

Der Injektorkörper stellt vorzugsweise eine Art Gehäuse für das Injektorsystem dar. Das Injektorsystem kann einteilig oder mehrteilig sein. In einer bevorzugten Ausführungsform der Erfindung werden der Injektorkörper, die Kanüle und/oder das Magazin durch einzelne Module bereitgestellt. Diese bilden dann in einem zusammengesetzten Zustand eine Funktionseinheit, den Injektor. Magazin und Kanüle können dabei auch durch ein einzelnes Bauteil, das als Kartusche bezeichnet wird, bereitgestellt werden. Das Magazin stellt einen Bereich oder eine Linsenkammer dar zum Aufbewahren oder Lagern der Linse und gegebenenfalls zum Laden des Injektors. In einer bevorzugten Ausgestaltung der Erfindung wird das Magazin als ein separates Modul bereitgestellt. In dem Magazin ist eine Linse gelagert. Vorzugsweise ist das Magazin, insbesondere zum Laden des Injektorsystems mit einer Linse, derart an der Kanüle positionierbar oder auf die Kanüle aufschiebbar, dass die Kanüle zumindest abschnittsweise im Inneren des Magazins oder an dem Magazin angeordnet oder befestigt ist. Der Aufnahmebereich für die Linse in dem Magazin zeichnet sich dadurch aus, dass die Linse dort .im Wesentlichen spannungsfrei, vorzugsweise eben, gelagert bzw. angeordnet ist. Vorzugsweise weist das Magazin einen Austrittsbereich bzw. eine Ausgangsöffnung auf, der bzw. die so ausgerichtet zu der Eingangsöffnung in dem Transportkanal angeordnet ist, dass die Linse mittels des Faltkörpers aus dem Magazin über Austrittsbereich bzw. die Ausgangsöffnung in den Transportkanal einschiebbar ist. In dieser Variante kann die Kanüle direkt in das in einem vorzugsweise separaten Behältnis befindliche Magazin eingeführt werden und zum Beispiel mit oder in diesem verrasten, so dass eine Funktionseinheit hergestellt wird. Vorzugsweise wird bzw. werden das Magazin und/oder die Linse in dem Behältnis steril gelagert.

Im Bereich der Erfindung liegt auch ein Magazin als solches für das vorstehend beschriebene Injektorsystem. Es ist ein Magazin mit einem Aufnahmebereich für wenigstens eine Linse, die in dem Aufnahmebereich mittels einer Halterungfestsetzbar ist, wobei die Halterung an der Seite, die der Linse zugeordnet ist, eine Rückhalteeinrichtung für eine Linse und/oder eine Verdrehsicherung für eine Linse aufweist.

In einer Ausführungsform wird das Injektorsystem mit dem Injektorkörper, dem Schieber und der Kanüle bereitgestellt. Das Magazin wird hierbei derart bereitgestellt, dass es auf die Kanüle des Injektors, insbesondere zum Laden des Injektorsystems mit einer Linse, so aufschiebbar ist oder so mit der Kanüle verbindbar ist, dass die Kanüle zumindest abschnittsweise im Inneren des Magazins oder am Magazin angeordnet ist. In einer weiteren Ausführungsform wird das Injektorsystem mit dem Injektorkörper und dem Schieber bereitgestellt. Das Magazin wird dann mit einer Kanüle bereitgestellt, so dass die aus' dem Magazin und der Kanüle gebildete Kartusche mit dem Injektorkörper verbindbar ist zum Laden des Injektors mit einer Linse.

Das vorstehend beschriebene In ektorsystem, - insbesondere die einzelnen Module zum Aufbau des Injektorsystems, wird bzw. werden mittels Spritzguss hergestellt. Um die Linse bzw. die Bewegung der Linse in dem Injektorsystem besser überwachen zu können, wird vorzugsweise ein transparenter Kunststoff verwendet. Ein Beispiel stellt ein Thermoplast dar . Der erfindungsgemäße Injektor ist insbesondere geeignet für alle weichen, faltbaren Intraokularlinsen. Diese sind zum Beispiel aufgebaut aus Acryl, Silikon und/oder Hydrogel. Der erfindungsgemäße Injektor ist leicht anpassbar an unterschiedliche Linsentypen, insbesondere hinsichtlich deren Aufbau und/oder deren Material.

Die konkreten Abmessungen und/oder Formen der einzelnen Module und/oder der Merkmale des Injektorsystems sind unter anderem abhängig von dem Design einer zu implantierenden Intraokularlinse. Der erfindungsgemäße Injektor kann als vorgeladener Wegwerfoder Einweg-Injektor verwendet werden.

Im Allgemeinen können die folgenden Größenordnungen vorliegen:
Der Injektorkörper besitzt eine Länge von etwa 50 mm bis etwa 70 mm und/oder einen Durchmesser (ohne Griffe) von etwa 8 mm bis etwa 16 mm.

Der Schieber besitzt eine Länge (ohne Griff) von etwa 100 mm bis etwa 130 mm und/oder einen Durchmesser von etwa 6 mm bis etwa 8 mm im ersten Abschnitt und von etwa 1 mm bis etwa 3 mm im zweiten Abschnitt.

Die Kanüle besitzt eine Länge von etwa 45 mm bis etwa 65 mm und/oder einen Durchmesser von etwa 5 mm bis etwa 15 mm im Bereich der Eingangsöffnung. Der Transportkanal hat im Bereich der Eingangsöffnung einen Durchmesser von etwa 3 mm bis etwa 7 mm und/oder im Bereich der Austrittsöffnung einen Durchmesser von etwa 1 bis 3 mm.

Das Magazin besitzt eine Länge von etwa 25 mm bis etwa 35 mm und/oder eine Höhe von etwa 10 mm bis etwa 20 mm und/oder eine Breite von etwa 5 mm bis etwa 15 mm.

Die vorliegende Erfindung wird anhand der nachfolgenden Ausführungsbeispiele im Einzelnen erläutert. Hierzu wird auf die beigefügten Zeichnungen Bezug genommen. Die gleichen Bezugszeichen in den einzelnen Zeichnungen beziehen sich auf die gleichen Teile.

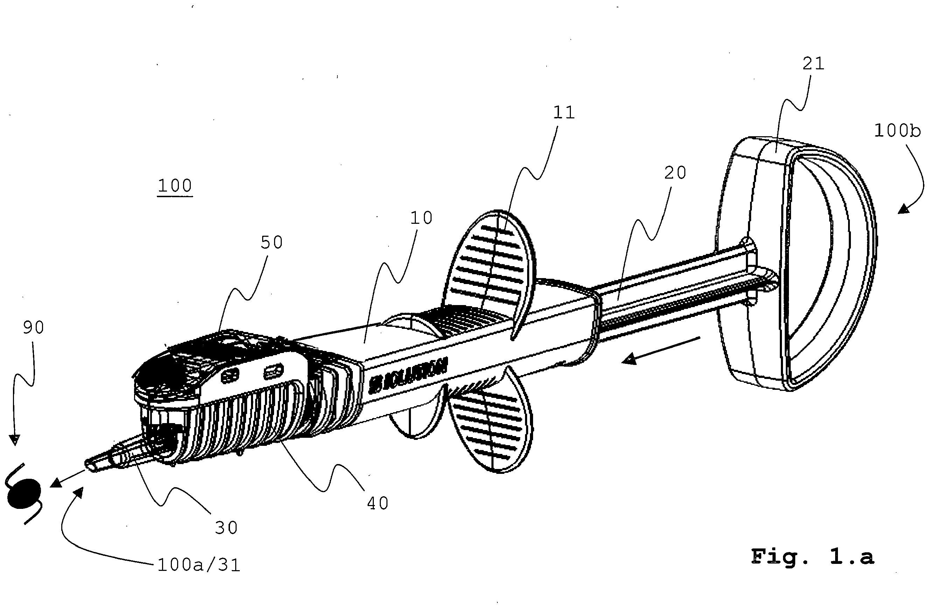

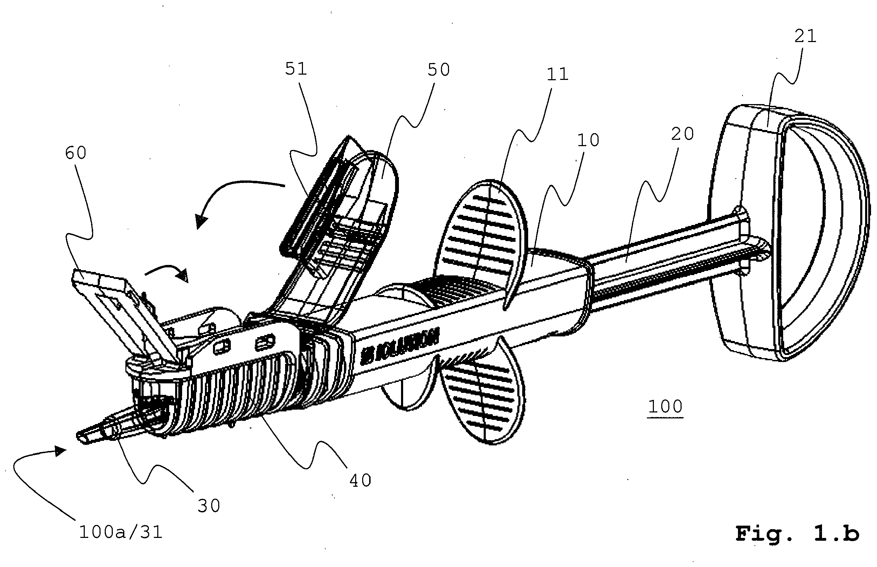

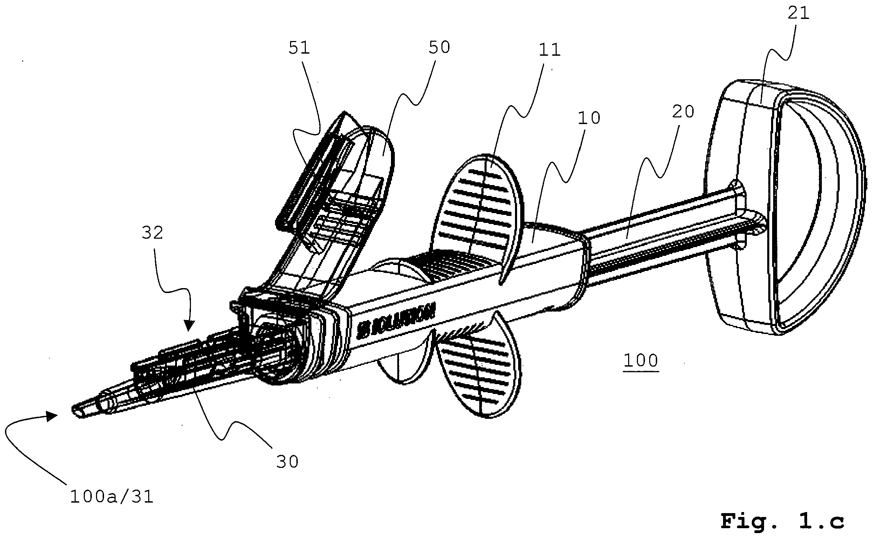

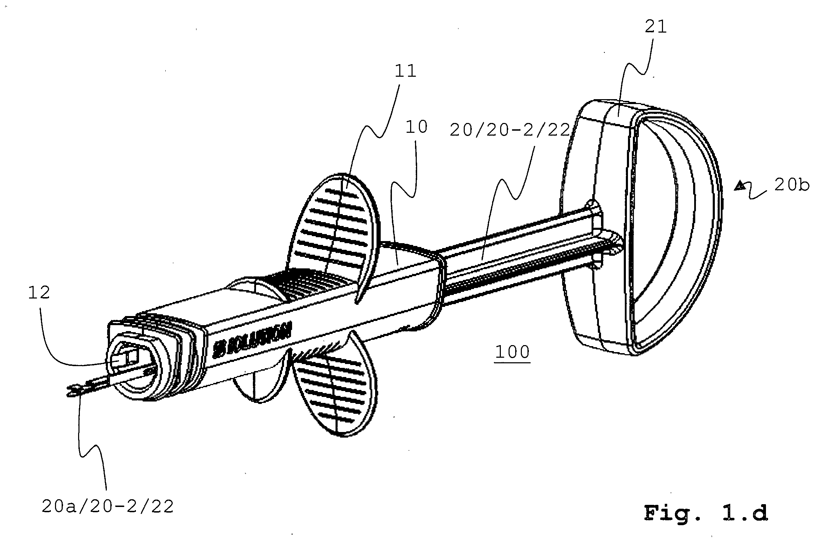

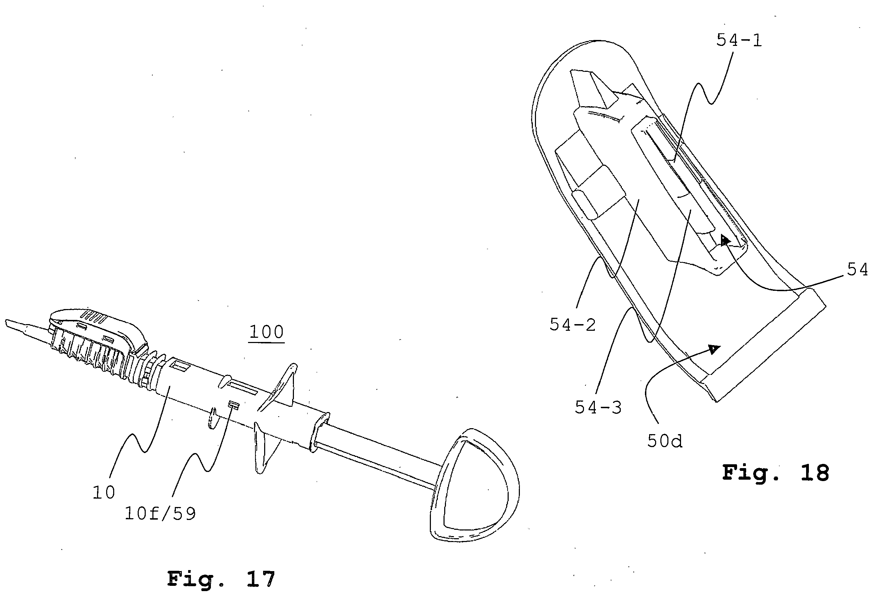

Fig. 1.a bis Fig. 17 zeigen verschiedene Ausführungsbeispiele von Injektoren und Fig. 18 zeigt einen Injektor mit den erfindungsgemäßen Merkmalen.
Fig. 1.a bis 1.d zeigen einen Injektor im zusammengebauten Zustand (Fig 1.a), mit offener Halteklappe und offener Faltklappe und daher mit nicht eingeführter Faltrippe (Fig. l.b), ohne Magazin (Fig. 1.c) und ohne Kanüle (Fig 1.d)..
Fig. 2.a und 2.b illustrieren in einer perspektivischen Ansicht das Bestücken des Magazins mit einer Linse mit offener (Fig. 2.a) und geschlossener (Fig. 2.b) Halteklappe.
Fig. 3.a bis 3.d illustrieren in einer perspektivischen Außenansicht das Laden des Injektors (Fig. 3.a bis 3.c) und das Entsichern des Injektors (Fig. 3. d).
Fig. 4.a bis 4.c illustrieren in einer perspektivischen Innenansicht das Bestücken des Magazins (Fig. 4. a), das Entsichern des Injektors (Fig. 4.b) und das Ausstoßen der Linse aus dem Injektor (Fig.4.c) .
Fig. 5.a und 5.b zeigen in einer perspektivischen Ansicht die Anordnung der Linse in ihrem nicht gefalteten Zustand in der Ausgangsposition im Magazin (Fig. 5. a) sowie in ihrem gefalteten Zustand in der Kanüle (Fig. 5.b) .
Fig. 6.a bis 6.c zeigen eine Detailansicht des Gehäuses in einer perspektivischen Außenansicht (Fig. 6.a), im Querschnitt (Fig. 6.b) und in einer Ansicht auf die Rückseite (Fig. 6.c).
Fig. 7.a bis 7.c zeigen eine Detailansicht des Schiebers in einer perspektivischen Ansicht (Fig. 7.a), in einer Seitenansicht (Fig. 7.b) und in einer vergrößerten Seitenansicht auf seine Spitze (Fig.7. c). Fig. 8.a bis 8.f zeigen eine Detailansicht des Magazins in einer perspektivischen Ansicht (Fig. 8.a + 8.b), in einer Seitenansicht (Fig. 8.c), einer Ansicht auf die Vorderseite des Magazins (Fig. 8.d) und in einer perspektivischen Ansicht mit Linse auf die Unterseite de-r Halteklappe (Fig. 8.e und8. f),
Fig. 9.a bis 9.c zeigen eine Detailansicht der Kanüle in einer perspektivischen Ansicht (Fig. 9.a), in einer Ansicht auf die Vorderseite (Fig. 9.b) und in einem Querschnitt (Fig. 9..c).
Fig. 10. a und 10. b zeigen eine alternative Ausführungsform mit einer Kartusche in einer perspektivischen Ansicht (Fig. 10. a) und in einem Querschnitt (Fig. 10. b) .
Fig. 11 zeigt eine Ausführungsform des erfindungsgemäßen Injektors mit einem modifizierten Faltkörper und einem Haptikschieber in einer perspektivischen
Ansicht Fig. 12. a und 12. b zeigen den modifizierten Faltkörper und den Haptikschieber aus Figur 11 ohne die weiteren Injektorbauteile in zwei seitlichen Ansichten. Fig. 13. a bis 13. d zeigen den modifizierten Faltkörper aus Figur 11 in unterschiedlichen Ansichten.
Fig. 14 illustriert eine erste Ausführungsform des Haptikschiebers aus den Figuren 11 bis 12. b.
Fig. 15 illustriert eine zweite Ausführungsform eines Haptikschiebers .
Fig. 16. a und 16. b zeigen den Haptikschieber aus Figur 15 in Wirkverbindung mit dem Linsenschieber und dem Faltkörper in einer Aufsicht (Fig. 16. a) und in einer Unteransicht (Fig. 16. b).
Fig. 17 zeigt eine Ausführungsform des erfindungsgemäßen Injektors mit dem Haptikschieber aus Figur 15.
Fig. 18 zeigt die erfindungsgemäße Ausführungsform eines Faltkörpers mit einen Aufnahmebereich für die hintere Haptik einer Linse.

### Detaillierte. Beschreibung der Erfindung:

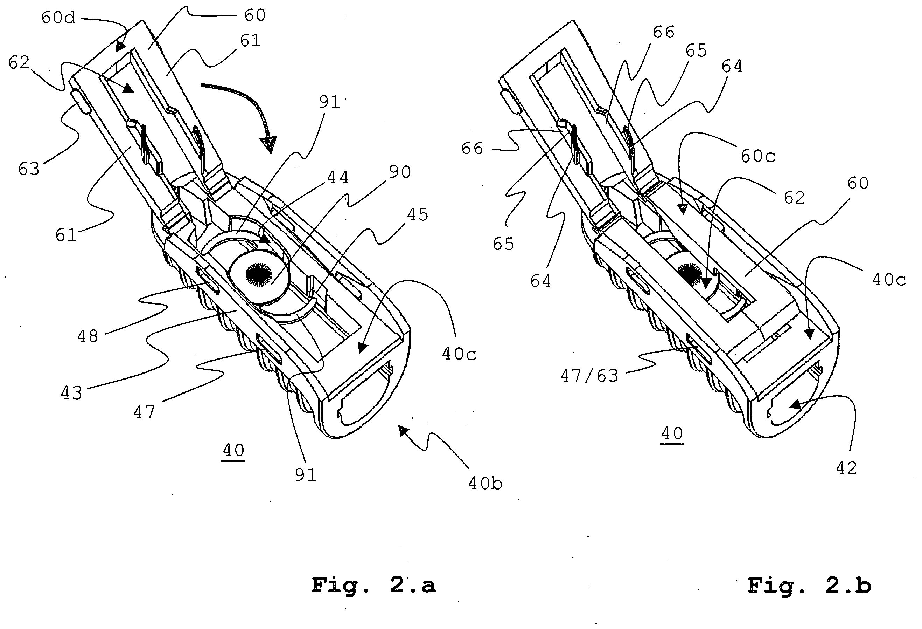

Ein Injektorsystem 100 wird am Beispiel eines modular aufgebauten Injektors 100 beschrieben, bei welchem die Halterung 60 als eine Klappe 60 ausgebildet ist und bei dem die Faltplatte 51 an einer Klappe 50 angebracht ist. Die als eine Klappe 60 ausgeführte Halterung 60 wird nachfolgend als Halteklappe 60 bezeichnet. Die Klappe 50, welche die genannte Faltrippe 51 trägt, wird nachfolgend als Halteklappe 60 bezeichnet. Der Injektorkörper 10 wird nachfolgend als Gehäuse 10 bezeichnet. Die Figuren 1.a bis 1.d zeigen einen Injektor 100 in seinem zusammengebauten Zustand. Die Module zum Aufbau des Injektors 100 umfassen ein Gehäuse 10, einen Schieber 20, eine Kanüle 30 und ein Magazin 40. Details zu den einzelnen' Modulen 10 bis 40 sind in den nachfolgenden Figuren 2.a bis 10. b beschrieben.

Figur 1.a zeigt den vollständig zusammengebauten Injektor 100. Die Kanüle 30 ist an der Vorderseite 10a des Gehäuses 10 angeordnet. Die Kanüle 30 und das Gehäuse 10 sind zum Beispiel einrastend miteinander verbunden. Die Kanüle 30 - hat einen Transportkanal 31 für die Linse 90 zum Ausbringen der Linse 90 aus dem Injektor 100 bzw. zum Einbringen der Linse 90 in ein Auge. Auf der Kanüle 30 ist das Magazin 40 positioniert, vorzugsweise aufgesteckt. Vorzugsweise sind die Kanüle 30 und das Magazin 40 einrastend miteinander verbunden.

Die Faltklappe 50, welche die Faltrippe 51 trägt, befindet sich im heruntergeklappten Zustand. Die Linse 90 befindet sich dadurch bereits im Transportkanal 31 der Kanüle 30.

Der Injektor 100 ist sozusagen entsichert. Der Injektor 100 ist betriebsbereit. Der Schieber 20 wird über die Rückseite 10b des Gehäuses 10 eingeführt. Er ist axial verschiebbar in dem Gehäuse 10 angeordnet. Der Schieber 10 ist in dem Gehäuse 10 so angeordnet, dass er aus der Vorderseite 10a des Gehäuses 10 so austritt, dass er in den Transportkanal 31 der Kanüle 30 eintreten kann. Der Schieber 10 und der Transportkanal 31 sind so in ihrer Form und/oder ihrer Größe aufeinander angepasst, dass der Schieber 20 auch indem Transportkanal 31 axial verschiebbar angeordnet ist. Über ein Verschieben bzw. Drücken des Schiebers 20 in Richtung der Vorderseite 100a des Injektors 100 wird die Linse 90, welche sich im Transportkanal 31 der Kanüle 30 befindet, aus dem Injektor 100 ausgestoßen.

Die Vorderseite 30a der Kanüle 30 oder des Transportkanals 31 definiert die Austrittsöffnung für die Linse 90. Der Schieber 20 bzw. der Injektor 100 kann beispielsweise betätigt werden, indem der Zeigefinger und der Mittelfinger an den Griffen 11 des Gehäuses 10 anliegen und der Daumen in den Griff 21 des Schiebers 20 eingreift. Für weitere Details zum Laden bzw. Bestücken, Sichern, Entsichern und/oder Anwenden des Injektors 100 bzw. Ausstoßen der Linse sei auf die Ausführungen zu den Figuren 2.a bis 5.b verwiesen.

Figur 1.b zeigt den Injektor 100 in einem nicht geladenen bzw. bestückten Zustand. Es befindet sich noch keine Linse 90 in dem Magazin 40. Das Magazin 40 ist mit geöffneter Halteklappe 60 dargestellt. Dementsprechend ist auch die Faltklappe 50 in einem nicht heruntergeklappten Zustand dargestellt. Das Magazin 40 kann zum Beispiel erst dann mit einer Linse 90 bestückt werden, wenn es bereits an dem Injektor 100 angeordnet ist bzw. auf der Kanüle 30 aufgesetzt ist.

Vorzugsweise wird das Magazin 40 jedoch mit einer Linse 90 bestückt und die Linse 90 mit der Halteklappe 60 im Magazin 60 befestigt, bevor das Magazin 40 auf den Injektor 100 bzw. die Kanüle 30 aufgezogen wird.

Dies ist insbesondere vorteilhaft, da die Bestückung unter sterilen Bedingungen, zum Beispiel von einem Hersteller für Linsen, durchgeführt werden kann. Das bestückte Magazin 40 kann dann in einem Aufbewahrungsbehälter, beispielsweise in einem Blister, welcher vorzugsweise mit einer sterilen Flüssigkeit befüllt ist, steril gelagert werden.

Vor der Inbetriebnahme des Injektors 100 wird zunächst der Aufbewahrungsbehälter geöffnet. Das Magazin 40 kann auf die Kanüle 30 aufgezogen werden, indem der Injektor 100 in den Aufbewahrungsbehälter eintaucht und das Magazin 40 auf die Kanüle 30 aufgesteckt wird. Dies bietet die Vorteile, dass die sterilen Bedingungen möglichst lange erhalten bleiben und dass der Transportkanal 31 der Kanüle 30 benetzt wird mit der Aufbewahrungsflüssigkeit, so dass die Linse 90 und der Schieber 20 besser in dem Transportkanal 31 gleiten können.

Der Injektor 100, insbesondere das Gehäuse 10, der Schieber 20, die Kanüle 30 und/oder das Magazin 40 kann bzw. können auch mehrfach verwendet werden. Vorzugsweise werden diese aber als Einwegbauteile verwendet.

Um einen verbesserten Überblick über den Aufbau bzw. den modularen Charakter des Injektors 100 zu erhalten-, ist der Injektor 100 in Figur 1.c ohne das Magazin 40 dargestellt. Vorzugsweise sind der so dargestellte Injektor 100 (ohne das Magazin 40) und das Magazin 40 getrennt, insbesondere jeweils unter sterilen Bedingungen, verpackt, in einer Verpackung für den Injektor und in einer Verpackung für das insbesondere bestückte Magazin. Vorzugsweise werden die beiden Verpackungen kurz vor der Inbetriebnahme des Injektors 100 geöffnet, der Injektor 100 der Verpackung entnommen und, wie beispielhaft vorstehend ausgeführt, mit dem Magazin 40 verbunden. Zum besseren Verständnis ist der Injektor 100 in Figur 1.d auch noch ohne die Kanüle 30 dargestellt. Die Kanüle 30 und das Gehäuse 10 können beispielsweise zusammengefügt oder verbunden sein mittels eines Rastmechanismus und/oder durch Verschrauben.

Um die Funktion des Injektors 100 bzw. das Zusammenwirken der Halteklappe 60 und der Faltklappe 50 zu verdeutlichen, zeigen die Figuren 2.a bis 5.b die Positionierung der Linse 90 in einem nicht gefalteten im Magazin 40 sowie in einem gefalteten Zustand im Transportkanal 31.

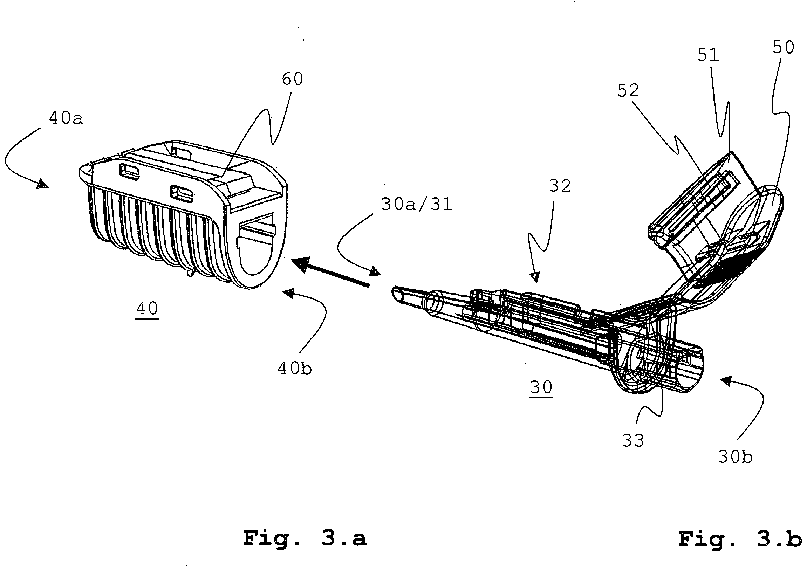

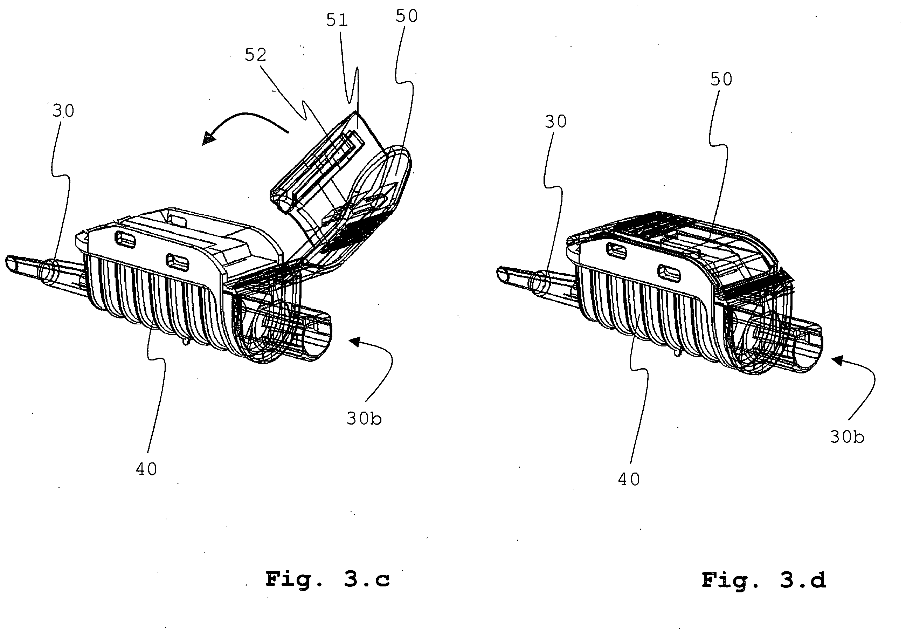

Zunächst illustrieren die beiden Figuren 2.a und 2.b die Bestückung des Magazins 40. Gezeigt ist eine Detailansicht eines mit einer Linse 90 geladenen bzw. bestückten Magazins 40 mit geöffneter (Fig. 2.a) und geschlossener Halteklappe 60 (Fig. 2.b). Das Magazin 40 wird durch einen hohlen Körper bereitgestellt. Im Inneren des Magazins 40 befindet sich ein Hohlraum 41, der zu einer Oberseite 40c des Magazins 40 und zu einer Vorderseite 40a sowie zu einerRückseite 40b des Magazins 40 offen ist und zur Unterseite 40d durch den Boden 42 und zur Seite durch die Seitenwände 43 begrenzt ist. Es wird dadurch in dem Magazin 40 eine axiale Durchgangsöffnung als Aufnahmebereich für die Kanüle 30 gebildet, in welchen die Kanüle 30 einführbar ist (siehe dazu auch die Figuren 3.a und 3.b).

Im Innenraum 41 des Magazins 40 ist ein Aufnahmereich 44 für die Linse 90 und gegebenenfalls für die Linsenhaptik 91 vorgesehen. Der Aufnahmebereich 44 für die Linse 90 ist als wenigstens eine Aussparung 44 in einer Innenseite der Wand 43 des Magazins 40 ausgeführt. Im Detail wird hier der Aufnahmebereich 44 durch zwei Aussparungen 44 in der Innenseite der Wand 43 des Magazins 40 gebildet. Die zweiAussparungen 44 sind in den gegenüberliegen Wänden 43 des Magazins.40 eingebracht. Aufgrund der gewählten Darstellung ist in Figur 2.a nur eine Aussparung 44 sichtbar. Die Aussparungen 44 sind an die Form und Größe der Linse 90 und gegebenenfalls ihrer Haptik 91 angepasst. Vorzugsweise sind sie zumindest abschnittsweise gekrümmt. Die Linse 90 und/oder die Haptik 91 liegt bzw. liegen zumindest abschnittsweise auf dem Boden der jeweiligen Aussparung 44 auf. Der Boden der Aussparung 44 liegt oberhalb des Transportkanals 31 und unterhalb der Oberseite 40c des Magazins.

Insbesondere um ein Verdrehen oder Verklemmen der Haptiken 90 zu verhindern, sind noch zwei Führungsbereiche 45 für die Haptiken 91 vorgesehen. Diese sind als Aussparungen 45 oder Kanal 45 in der Innenseite der Wand 43 des Magazins 40 ausgebildet. Diese haben hier keinen Boden. Sie erstrecken sich bis zu der Durchgangsöffnung, in welche die Kanüle 30 bzw. der Transportkanal 31 eingeführt wird. Die Führungsund/oder Aufnahmebereiche 45 für die Haptiken 90 sind entlang der Längsachse des Magazins 40 seitlich versetzt zu den Aufnahmebereichen 44 angeordnet.

Um einen sicheren Transport der Linse 90 in dem Magazin 40 zu ermöglichen und beispielsweise ein Herausfallen derLinse 90 zu verhindern, ist die Halteklappe 60 vorgesehen. Die Halteklappe 60 ist hier im Wesentlichen U-förmig gewählt. Die Halteklappe 60 weist die zwei Schenkel 61 und eine zwischen den Schenkeln 61 gebildete Öffnung 62 auf. Die Öffnung 62 stellt einen Durchgangsbereich für die Faltklappe 51 bereit. Die Halteklappe 60 ist hier mit dem Magazin 40 verbunden. Sie ist drehbar mit dem Magazin 40 verbunden. Über die beiden Schenkel 61 ist die Halteklappe 60 mit dem Magazin 40 verbunden. Vorzugsweise sind die Halteklappe 60 und das Magazin 40 einteilig ausgeführt. Durch eine entsprechend dünn dimensionierte Verbindung zwischen der Halteklappe 60 und dem Magazin 40 wird eine Art Gelenk zwischen der Halteklappe 60 und dem Magazin 40 gebildet. Durch ein Herunterschwenken der Halteklappe 60 wird die Linse 90 in dem Magazin 40 in ihrer Position festgesetzt (siehe Figur 2.b). Aus Darstellungsgründen ist die Halteklappe 60 in Figur 2.b sowohl in ihrer geöffneten als auch in ihrer geschlossenen Stellung eingezeichnet.

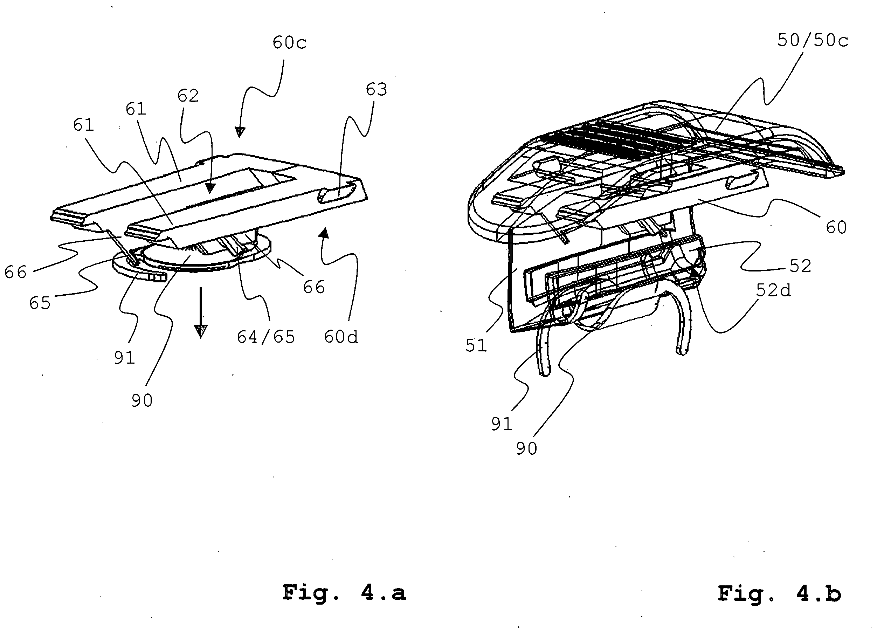

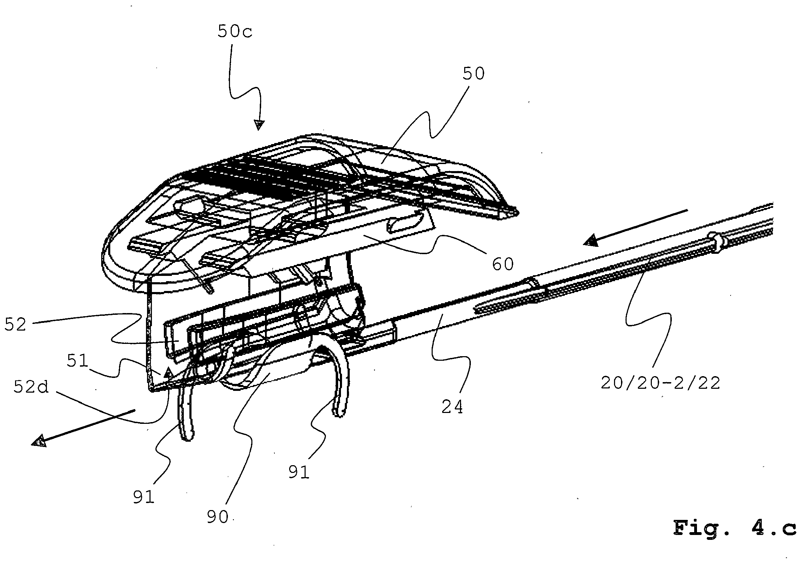

An der Unterseite 60d der Halteklappe 60 sind die Mittel zum Positionieren oder Arretieren der Linse 90 angeordnet (siehe Figur 2.a). Diese umfassen eine Rückhalteeinrichtung 64 und eine Verdrehsicherung 65. Für weitere Details zu den Mitteln sei auf die Ausführungen zu den Figuren 4.a und 8.a bis 8.f verwiesen.

Die Mittel zum Arretieren von Halteklappe 60 und Magazin 40, im heruntergeschwenkten Zustand der Halteklappe 60, sind im Wesentlichen basierend auf einem Rastmechanismus. Dazu sind an der Außenseite der Halteklappe 60 vorzugsweise zwei Vorsprünge 63 vorgesehen, welche, in einem heruntergeklappten Zustand, in zwei korrespondierende Aussparungen 47 oder Öffnungen 47 in der Magazinwand 43 eingreifen (siehe Figur 2.b). Die Vorsprünge 62 der Halteklappe 60 sind in die Öffnungen 47 des Magazins 40 eingerastet.

Seitlich versetzt zu den Öffnungen 47 sind noch zwei weitere Öffnungen 48 angeordnet. Sie sind Teil der Befestigungs- oder Arretiermittel für die Faltrippe 51. Dazu sei auf die Ausführungen zu Figur 3.d verwiesen.

Figuren 3.a bis 3.d illustrieren das Laden des Injektors 100. Zur besseren Übersicht sind das Gehäuse 10 und der Schieber 20 nicht dargestellt. Das Magazin 40 ist mit einer Linse 90 bestückt. Die Halteklappe 60 ist geschlossen bzw. im heruntergeklappten Zustand und vorzugsweise einrastend mit dem Magazin 40 verbunden (siehe Figur 3.a). Die Linse 90 befindet sich in den Aufnahmebereichen 44 des Magazins 40 und wird durch die Halteklappe 60 bzw. durch die Mittel zum Arretieren dort festgesetzt. Die Mittel zum Arretieren werden zumindest durch die Rückhalteeinrichtung 64 und/oder die Verdrehsicherung 65 bereitgestellt. Die Linse 90 befindet sich in ihrer Ausgangsposition, die insbesondere geeignet ist zum Transportieren des Magazins 40. Das Magazin 40 wird bereitgestellt. Das Festsetzen der Linse e 90 durch die Mittel ist zum Beispiel in Figur 4.a illustriert. Dort sind nur die Linse -90 und die Halteklappe 60 als ein Bestandteil des Magazins 40 dargestellt.

In einem nachfolgenden Schritt wird das Magazin 40 mit dem Injektor 100, vorzugsweise einrastend, verbunden (siehe dazu die Figuren 3.a und 3.b und den dazwischen liegenden Pfeil in Kombination). Im Detail wird das Magazin 40 auf die Kanüle 30 aufgesteckt bzw. wird die Kanüle 30 in das Magazin 40 eingeführt. Die Kanüle 30 wird dazu mit ihrer Spitze bzw. Vorderseite 30a über die Rückseite 40b des Magazins in das Magazin 40 eingeführt. Das Magazin 40 ist als eine Art Kragen auf der Kanüle 30 angeordnet (siehe dazu Figur 3.c) . Der Injektor 100 befindet sich nun in einem geladenen bzw. bestückten Zustand. Die Faltklappe 50 mit der Faltrippe 51 befindet sich in einem geöffneten bzw. nicht heruntergeklappten Zustand. Die Faltklappe 50 ist hier mit der Kanüle 30 verbunden. Sie ist drehbar mit der Kanüle 30 verbunden. Vorzugsweise sind die Faltklappe 50 und die Kanüle 30 einteilig ausgeführt. Durch eine entsprechend dünn dimensionierte Verbindung zwischen der Faltklappe 50 und der Kanüle 30 wird eine Art Gelenk zwischen der Faltklappe 50 und der Kanüle 30 gebildet. In einem nächsten Schritt wird der Injektor 100 entsichert. Dazu wird die Linse 90 von ihrer Ausgangsposition in dem Magazin 40 in ihre Transportposition in der Kanüle 30 bzw. im Transportkanal 31 bewegt bzw. überfuhrt. Die genannte Transportposition kennzeichnet die Position, von der aus die Linse 90 mittels des Schiebers 20 aus dem Injektor 100 ausgestoßen und in ein Auge eingeführt werden kann. Dazu wird die Faltklappe 50 in Richtung auf die Oberseite 40c des Magazins 40 geklappt oder geschwenkt. Die an der Unterseite 50d der Faltklappe 50 angeordnete Faltrippe 51 greift zwischen den Schenkel 61 der Halteklappe 60 hindurch in das Magazin 40 ein. Die Faltklappe 51 kommt in Auflage auf die Oberseite der . Linse 90 und schiebt sie, indem die Linse 90 im Querschnitt U-förmig gefaltet wird, aus ihrer Ausgangsposition im Magazin 40 heraus in den Transportkanal 31 der Kanüle 30 hinein (siehe dazu Figur 4.b, in der nur die Halteklappe 60, die Faitklappe 50 und die Linse 90 dargestellt sind). Um ein sicheres Falten der Linse 90 und ein mögliches Verrutschen und Verklemmen der Linse 90 zu verhindern, sind an der Faltrippe 51 vorzugsweise zwei Rückhaltekanten 52 vorgesehen. Diese können auch am oberen Ende des Transportkanals 31 im Bereich der Eingangsöffnung 32 vorgesehen sein.

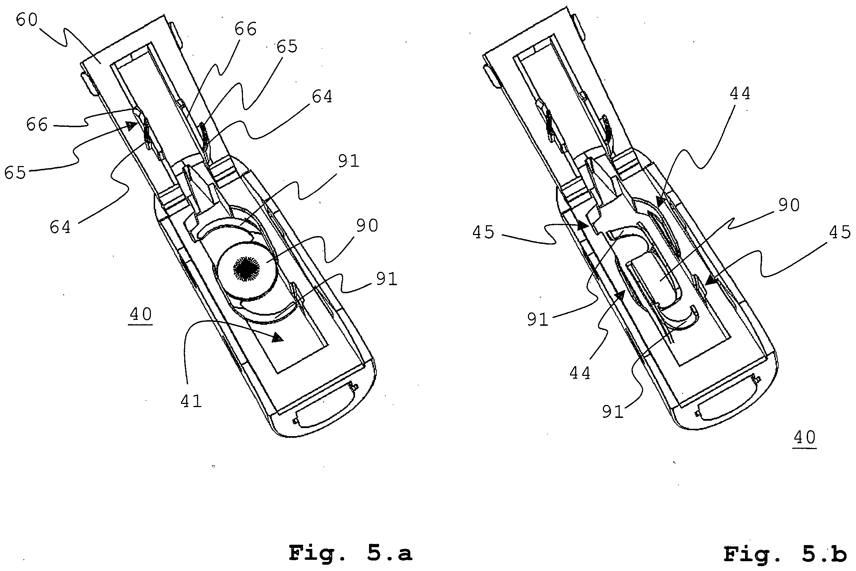

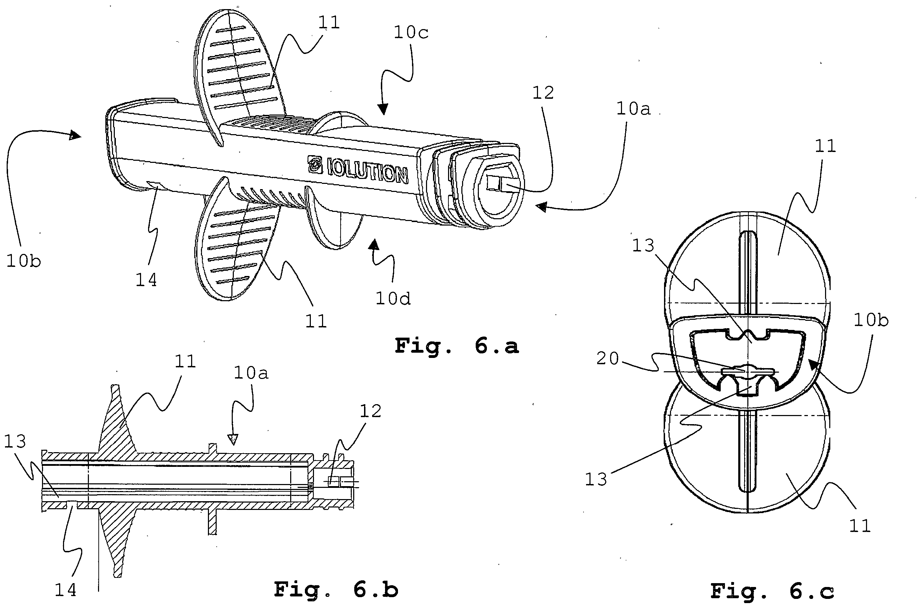

In einem nächsten Schritt wird die Linse aus dem Injektor 100 ausgebracht und in ein Auge eingeführt. Mittels des Schiebers 20 wird die Linse 90 aus dem Injektor 100 ausgeschoben (siehe dazu Figur 4.c). Figur 4.c entspricht im Wesentlichen der Figur 4.b. Zusätzlich ist jedoch noch der Schieber 20 dargestellt. Im Detail ist der vordere Abschnitt 20-2 des Schiebers 20 dargestellt. Um die Linse 90 mit dem Schieber 20 sicher erfassen und ein definiertes Ausbringen der Linse 90 aus dem Injektor 100 erzielen zukönnen, weist der Schieber 20 an seiner Spitze Führungsmittel 23 auf. Diese sind als eine Art Gabel 23 ausgeführt (siehe dazu auch die Figuren 6.a bis 6.c). Der Schieber 20 wird dabei an die Linse 90 herangeführt. Die Linse 90 kommt in Eingriff mit der Gabel 23, insbesondere zwischen die beiden Zacken der Gabel 23. Weiterhin weist der Schieber 20 noch einen Aufnahmebereich 24 für die oder eine Haptik 91 der Linse 90 auf. Der Aufnahmebereich 24 ist als eine Aussparung 24 oder Nut 24 in dem Arm 21 des Schiebers 20 ausgeführt. Die Haptik 91 legt sich in diese Aussparung 24 beim Ausbringen der Linse 90 ein. Dadurch kann ein Verklemmen und eine mögliche Beschädigung der Haptik 91 vermieden werden. Um noch einmal die Positionsänderung der Linse 90 bzw. den Übergang von der Ausgangsposition der Linse 90 in dem nicht gefalteten Zustand in die Transportposition der Linse 90 in dem gefalteten Zustand zu verdeutlichen, zeigen die Figuren 5.a und 5.b das Magazin 40 in einem geöffneten Zustand. Die Linse 90 ist in der Figur 5.a in ihrer Ausgangsposition angeordnet. In der Figur 5. b befindet sich die Linse 90 dagegen bereits in der Transportposition. Zum besseren Verständnis wurde auf eine Darstellung der Faltklappe 50 mit der Faltrippe 51, welche die Linse 90 von der Ausgangs- in die Transportposition bewegt bzw. verschiebt, als auch auf die Darstellung der Kanüle 30 verzichtet. Daher scheint es so, als ob die Linse 90 in der Figur 5.b auf dem Boden 42 des Magazins 40 aufliegt. In der Realität befindet sich die Linse 90 in dem Transportkanal 31 der Kanüle 30, welche in dem Innenraum des Magazins 40 aufgenommen ist.

Die Faltklappe 50 ist, vorzugsweise einrastend, mit dem Magazin 40 verbunden. Die Mittel zum Verbinden von

Faltklappe 50 und Magazin 40 basieren somit auf einem Rastmechanismus. Dazu sind an der Außenseite der Faltklappe 50 vorzugsweise zwei Vorsprünge 53 vorgesehen, welche, in einem heruntergeklappten Zustand, in zwei korrespondierende Aussparungen 48 oder Öffnungen 48 in der Magazinwand 43 eingreifen (siehe Figur 3.d).

Vorstehend sind der Injektor 100 in seiner Gesamtheit und seine Arbeitsweise erläutert. In der folgenden Beschreibung sind dagegen die einzelnen Module 10, 20, 30, 40, 50 und 60, insbesondere mit ihren wesentlichen strukturellen Merkmalen, erläutert.

Zunächst ist in den Figuren 6.a bis 6.c das Gehäuse 10 mit den Griffen 11, an denen die Finger angelegt werden können, dargestellt. Dadurch kann der Injektor 100 wie eine Spritze bedient werden. Die hier nicht dargestellte Kanüle 30 ist über die Vorderseite 10a mit dem Gehäuse 10 verbunden. Sie ist hier mittels eines Rastmechanismus mit dem Gehäuse 10 verbunden. Am Gehäuse 10 sind Aussparungen 12 vorgesehen, in die korrespondierende, an der Kanüle 30 positionierte Rastnasen 33 eingreifen. Der in den vorliegenden Figuren nicht dargestellte Schieber 20 wird über die Rückseite 10b in das Gehäuse 10 eingeführt. Um ein präzises Gleiten des Schiebers 20 in dem Gehäuse 10 gewährleisten zu können, sind im Inneren des Gehäuses 10 Führungsmittel 13 vorgesehen. Diese sind als Führungsschienen 13 ausgebildet. Die Schienen 13 sind hier beispielhaft an der Innenseite der Oberseite 10c als auch an der Innenseite der Unterseite lOd angeordnet. Das Loch 14 definiert eine Endposition für den Schieber 20.

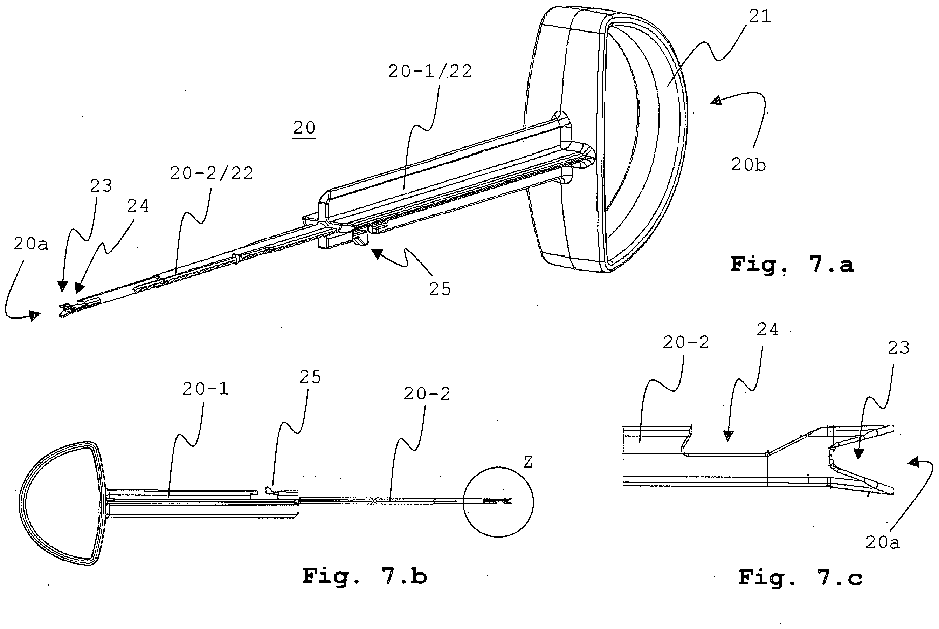

Als nächstes ist der Schieber 20 in den Figuren 7.a bis 7.c dargestellt. Er hat einen ersten Abschnitt 20-1 mit einer zu den Führungsschienen 13 im Gehäuse 10 korrespondierenden Form und Größe und ist hier beispielhaft kreuzförmig. Es werden dadurch Rippen zur Stabilisierung des Schiebers 20 gebildet. Zur Rückseite 20b hin ist an dem ersten Abschnitt 20-1 der Griff 21 angeordnet. Der erste Abschnitt 20-1 hat eine Länge, welche im Wesentlichen der Länge des Gehäuses 10 entspricht. Der erste Abschnitt 20-1 geht zur anderen Seite in einen zweiten Abschnitt 20-2 über. Dieser zweite Abschnitt 20-2 dringt zum Ausbringen der Linse 90 in den Transportkanal 31 der Kanüle 30 ein. Der zweite Abschnitt 20-2 besitzt eine zu dem Transportkanal 31 der Kanüle 30 korrespondierende Form und Größe und ist hier beispielhaft zumindest abschnittsweise kreuzförmig. Es werden dadurch Rippen zur Stabilisierung des Schiebers 20 gebildet. Der zweite Abschnitt 20-2 hat zur Vorderseite 20a des Schiebers 20 hin einen verjüngenden Querschnitt. Die Länge des zweiten vorderen Abschnitts 20-2 ist so gewählt, dass die Spitze des Schiebers 20 beim Ausbringen der Linse 90 aus dem Transportkanal herausragen kann. Der Schieber 20, der auch als Kolben 20 bezeichnet werden kann, ist vorzugsweise axial verschiebbar sowohl in dem Gehäuse 10 als auch in dem Transportkanal 31 in der Kanüle 30. Die Endposition des Schiebers 20 beim Ausstoßen der Linse 90 kann zum Beispiel, durch den Anschlag vom Griff 21 an der Rückseite 10b des Gehäuses 10 definiert sein.

Um die Linse 90 mit dem Schieber 20 sicher erfassen und ein definiertes Ausbringen der Linse 90 aus dem Injektor 100 erzielen zu können, weist der Schieber 20 an seiner Spitze oder Vorderseite 20a Führungsmittel 23 auf. Diese sind als eine Art Gabel 23 ausgeführt. Weiterhin weist der Schieber 20 noch einen Aufnahmebereich 24 für die oder eine Haptik 91 der Linse 90 auf. Der Aufnahmebereich 24 ist als eine Aussparung 24 im zweiten Abschnitt 20-2 des Schiebers 20 ausgeführt. Anstatt der Gabel 23 kann auch ein elastischesBauteil, zum Beispiel ein im Wesentlichen zylinderförmiger Puffer bzw. ein Zwischenstück, insbesondere aus Silikon, verwendet werden. Die Rastnase 25 greift beim Herausziehen des Schiebers 20 aus dem Gehäuse 10 in das Loch 14 in dem Gehäuse 10 ein, so dass dadurch eine Endposition für den Schieber 20 in dem Gehäuse" 10 definiert wird, so dass z.B. das Herausfallen des Schiebers 20 aus dem Gehäuse 10 verhindert werden kann.

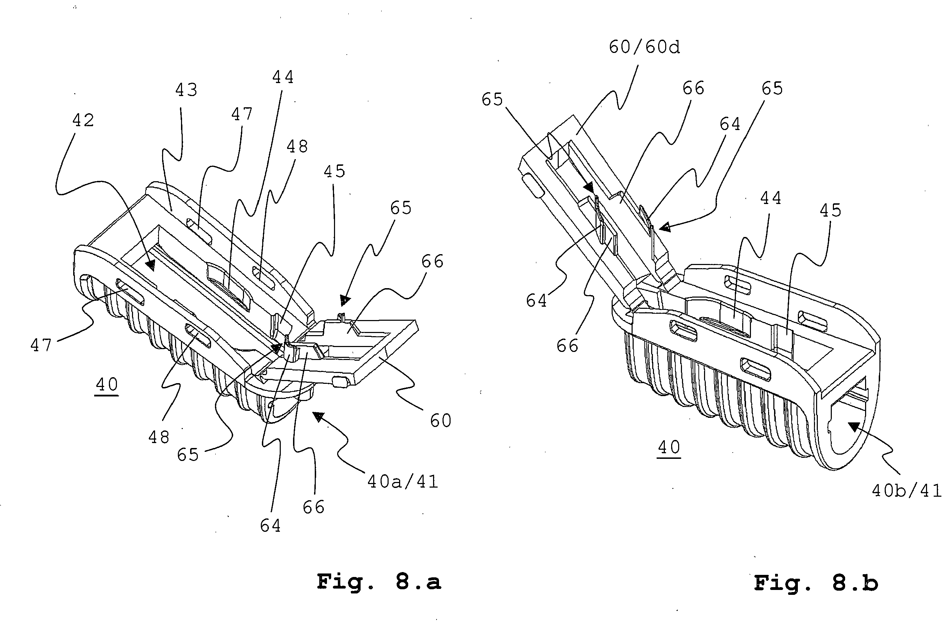

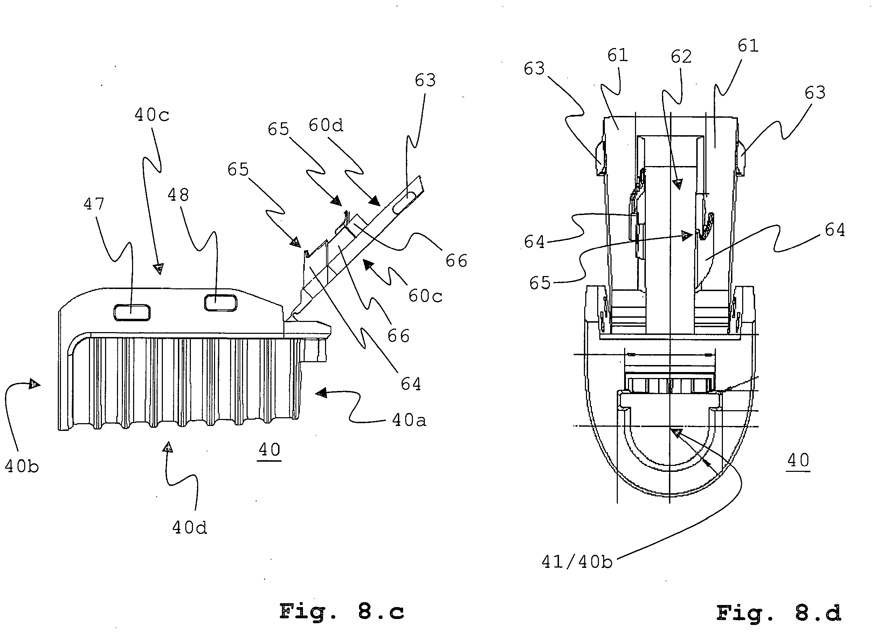

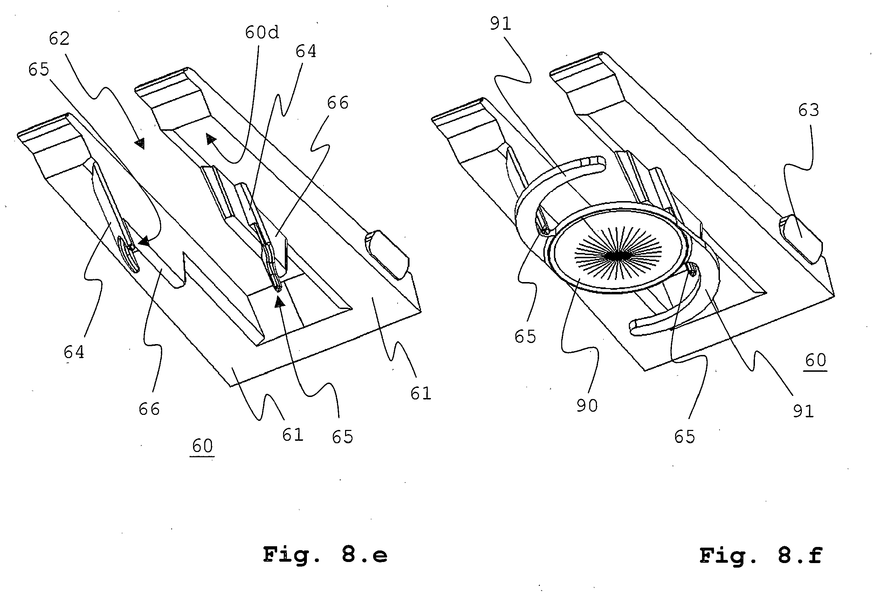

Die Figuren 8.a bis 8.d illustrieren das Magazin 40 mit der Halteklappe 60 ohne eine Linse 90. Die Figuren 8. e und 8.f zeigen dagegen nur die Halteklappe 60 mit eingelegter Linse 90, um die Wirkung Rückhalteeinrichtung 64 zusammen mit der Verdrehsicherung 65 besser illustrieren zu können.

Das Magazin 40 ist ein hohler Körper der zu den Seiten 40a und 40b und zur Oberseite 40c hin geöffnet ist. Das Magazin 40 ist eine Linsenkammer und insbesondere geeignet für den Transport und/oder die Aufbewahrung der Linse 90. Es weist einen Boden 42 und die Seitenwände 43 auf. Insbesondere um das Magazin 40 sicher greifen und halten zu können, ist an der Außenseite des Magazins 40 eine Vielzahl von Rippen angeordnet, die sich abschnittsweise über den Umfang des Magazins 40 erstrecken.

Im Inneren des Magazins 40 ist der Aufnahmebereich 44 für die Linse 90 und der Führungs- und/oder Aufnahmebereich 45 für die Haptiken 91 der Linse 45, insbesondere für das Armende der Haptiken 91, angeordnet. Der Auf ahmebereich 44 kann einen Abschnitt der Linse 90 und einen Abschnitt der Haptik 91, vorzugsweise den Ansatz der Haptiken 91 an der Linse 90, aufnehmen. Der Aufnahmebereich 44 für die Linse 90 und der Führungs- und/oder Aufnahmebereich 45 für die Haptiken 91 werden als jeweils als zwei Aussparungen 44 bzw. 45 in der Seitenwand 43 bereitgestellt. Die zwei Aussparungen 44 für die Linse 90 sind korrespondierend zu der Form und/oder der Größe der Linse 90 und gegebenenfalls der Haptik 91. Sie sind wenigstens abschnittsweise gekrümmt. Zudem besitzen sie hier einen Böden, auf dem ein Abschnitt der Linse 90 und gegebenenfalls der Haptik 91 zur Auflage kommen kann. Die zwei Aussparungen 44 sind axial versetzt zueinander angeordnet. Auch die zwei Aussparungen 45 sind axial versetzt zueinander angeordnet.

An dem Magazin 40 ist die Halteklappe 60 angeordnet. Zur Oberseite 40c hin ist das Magazin 40 mit der Halteklappe 60 verschließbar. Die Halteklappe 60 stellt eine Art Deckel für das Magazin 40 dar. Die Halteklappe 60 ist über seine zwei Rastnasen 63 einrastend mit den zwei Aussparungen 47 an dem Magazin 40 verschließbar. Zur Oberseite 40c hin nach oben versetzt (gegenüber den Aussparungen 47) sind noch zwei weitere Aussparungen 48 vorgesehen zum einrastenden Verbinden mit der hier nicht dargestellten Faltklappe 50.

Die Linse 90 wird durch die Klappe 60 gegen Herausfallen, Verrutschen und ein Verdrehen gesichert. Dazu sind an der Unterseite 60d der Halteklappe 60 Mittel zum Positionieren und/oder' Arretieren der Linse 90 vorgesehen. Die Mittel zum Positionieren und/oder Arretieren der Linse 90 umfassen hier eine Rückhalteeinrichtung 64 und eine Verdrehsicherung 65.

Die Rückhalteeinrichtung 64 wird durch zwei plattenförmige Vorsprünge 64 bereitgestellt, die jeweils an einem Schenkel 61 der Halteklappe 60 angeordnet sind. Diese sind zumindest abschnittsweise gekrümmt. Die Krümmung ist an die Krümmung einer Linse 90 angepasst. Der Radius der Krümmung ist dabei vorzugsweise derart, dass die plattenförmigen Vorsprünge 64 nicht wesentlich in die Optik der Linse 90 eingreifen. Sie sind vielmehr dem Rand der Linse 90 zugeordnet und hierbei insbesondere dem Bereich, wo sich der Ansatz der Haptik 91 an der Linse 90 befindet. Die gekrümmten plattenförmigen Vorsprünge 64 werden hier noch jeweils durch einen Träger 66 stabilisiert. Die zwei Träger 66 werden jeweils durch eine Art Rippe an der Unterseite 60d der Halteklappe 60 bereitgestellt. Die zwei Träger 66 dienen auch dazu, um die Halteklappe 60 zu versteifen. Der Vorsprung 64 kann sich an den Träger 66 anlehnen oder mit diesem einteilig sein. Die Verdrehsicherung 65 wird durch eine Art Nase 65 oder eine Art Zahn 65 an der Unterseite 60d der Halteklappe 60 bereitgestellt. Vorzugsweise wird sie durch zwei Nasen 65 bereitgestellt, welche jeweils an einem Schenkel 61 der Halteklappe 60 angeordnet sind. In der dargestellten Form sind die zwei Nasen 65 jeweils an einem plattenförmigen Vorsprung 64 angeordnet. Die Nasen 64 greifen ein in den Übergangsbereich zwischen Linse 90 und Haptik 91.

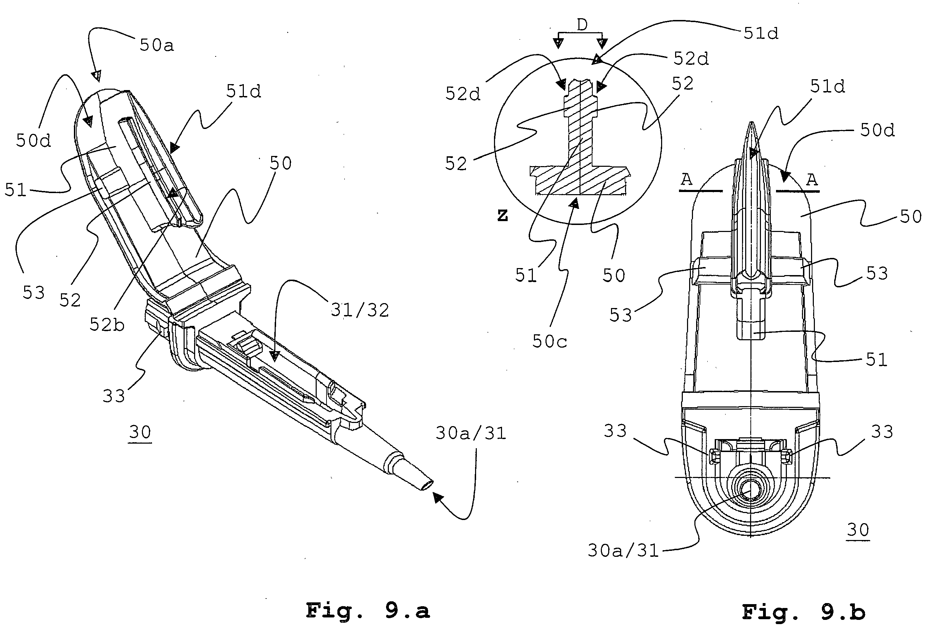

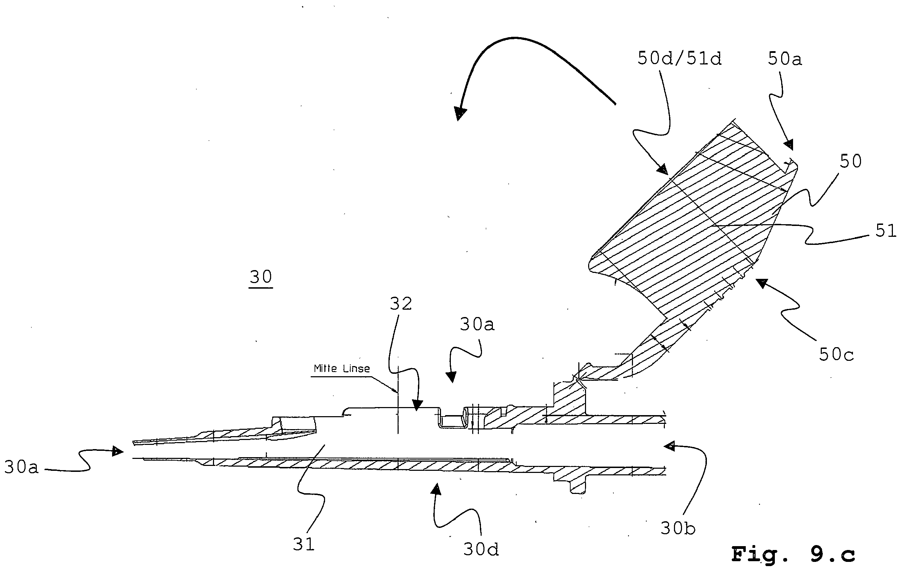

Die Figuren 9.a bis 9.c zeigen eine Kanüle 30. Die Kanüle 30 ist ein im Wesentlichen hohler Körper. Im Ihneren der Kanüle 30 befindet sich der Transportkanal 31 für den Transport der Linse 90 in das Auge. Die Kanüle 30 bzw. der Transportkanal 31 ist zu der Oberseite 30c hin zumindest abschnittsweise geöffnet mit der Öffnung 32. Der Transportkanal 31 ist zumindest im Bereich der Öffnung 32 zum Beispiel im Wesentlichen U-förmig.

Der Kanüle 30 ist die Faltklappe 50 zugeordnet. Diese ist schwenkbar an der Kanüle 30 angeordnet. Vorzugsweise sind die Kanüle 30 und die Faltklappe 50 einstückig. An der Unterseite 50d der Faltklappe 50 ist die Faltrippe 51 positioniert. Die Faltrippe 51 drückt beim Herunterklappen auf die Oberseite der Linse 90. Die Faltrippe 50 schiebt die Linse 90 aus ihrer Transportposition im Magazin 40 in die Transportposition im Transportkanal 31 und faltet bzw. rollt die Linse 90 gleichzeitig. Die Faltrippe 51 greift beim Herunterschwenken der Faltklappe 50 in die Öffnung 32 in dem Kanülenkörper 31 ein. Vorzugsweise hat die Faltrippe 51 eine zu der Form und/oder der Größe der Öffnung 32 in dem Transportkanal 31 im Wesentlichen korrespondierende Form und/oder Größe.

Die Faltrippe 51 hat einen in Richtung zur Vorderseite 50a der Faltklappe 50 hin verjüngenden Querschnitt. Die Breite der Faltrippe 51 nimmt zur Vorderseite 50a der Faltklappe 50 hin, vorzugsweise kontinuierlich, ab. Es wird eine Art Schneide an der Vorderseite der Faltrippe 51 gebildet. Die Schneide stellt eine Führung für das Gleiten der Linse 90 im Transportkanal 31 bereit und unterstützt das Falten bzw. Zusammenrollen der Linse 90 im -Transport kanal 31.

Oberhalb der unteren Kante der Faltrippe 51 ist auf jeder Seite der Faltrippe 51 wenigstens eine Rückhaltekante 52 angeordnet. Eine Rückhaltekante 52 ist als eine Art Steg oder Balken an der Faltrippe 51 ausgebildet. Sie erstreckt sich vorzugsweise nicht bis zu Vorderseite der Faltrippe 51 hin (siehe zum Beispiel Figur 9.a) . Die Rückhaltekanten 52 bieten mit ihrer Unterseite 52d eine Anlagefläche für die Linse 90 bzw. für die Linsekante. Die Strecke D zwischen der Unterseite 52d einer Rückhaltekante 52 zu der Unterseite der gegenüberliegenden Rückhaltekante 52 ist vorzugsweise im Wesentlichen korrespondierend mitdem Durchmesser der Linse 90. Beispielsweise ist die Länge D ungefähr gleich dem Durchmesser einer Linse 90. Dadurch kann ein Wegrutschen der Linse 90 beim Einschieben in den Transportkanal 31, das verbunden bzw. begleitet ist mit dem gleichzeitigen Falten der Linse 90, verhindert werden. Ohne die Rückhaltkanten 52 könnte die Linse 90 seitlich nach oben wegrutschen und verklemmen.

Die Unterseite 51d der Faltrippe 51 besitzt eine Vertiefung (siehe den vergrößerten Querschnitt Z entlang der Achse A-A in Figur 9.b). In der dargestellten Ausführungsform ist die. die Unterseite 51d zumindest abschnittsweise konkav. Damit kann die Linse 90 beim Einführen in den Transportkanal 31 besser durch die Faltrippe 51 erfasst werden.

Die Vorderseite 30a der Kanüle 30 bzw. die Spitze des Transportkanals 31 ist hier beispielhaft abgeschrägt.

Dadurch kann das sichere Ausbringen der Linse 90 aus dem Kanal 31 unterstützt werden.

Die Kanüle 30 wird über ihre Rückseite 30b mit dem Gehäuse , 10 verbunden. Dazu sind an der Kanüle 30 die Rastnasen 33 vorgesehen, die in am Gehäuse 10 ausgebildete Aussparungen 12, vorzugsweise rastend, eingreifen.

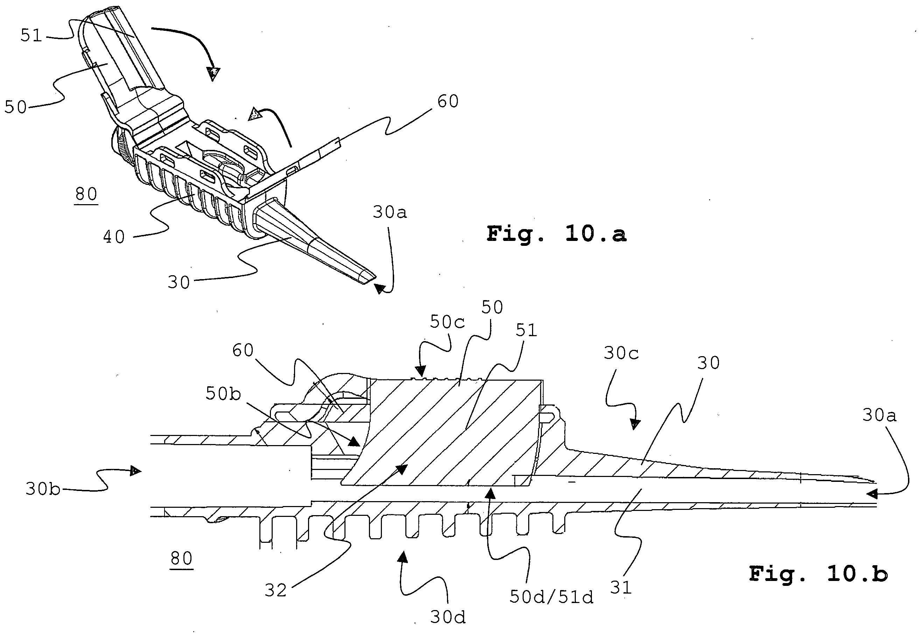

Die Figuren 10. a und 10. b zeigen eine alternative Ausführungsform mit einer Kartusche 80. Das Magazin 40 und die Kanüle 30 werden in dieser Ausgestaltung nicht durch zwei getrennte Module bereitgestellt, die durch ein Verbinden zu einer Funktionseinheit verbunden sind. DasMagazin 40 und die Kanüle 30 werden durch ein Bauteil, das hier als Kartusche 80 bezeichnet ist, bereitgestellt.Am Beispiel der Kartusche 80, insbesondere am Querschnitt in Figur 10. b, läßt sich noch einmal die Arbeitsweise des Injektors 100, insbesondere die Funktion der Halteklappe 60 und der Faltklappe 50 mit der Faltrippe 51, erläutern. Die hier beschriebenen Ausführungen lassen sich auch auf den insbesondere in den Figuren 8.a bis 9.c gezeigten modularen Aufbau übertragen: Um Wiederholungen zu vermeiden, wird für gleiche oder ähnliche Bestandteile auf die vorangegangenen Ausführungen zu dem Magazin 40 und der Kanüle 30 verwiesen.

Die Linse 90 ist zunächst mittels der heruntergeschwenkten Halteklappe 60 in der Kartusche 80 befestigt. Die Linse 90 befindet sich in ihrer Ausgangsposition, insbesondere im Wesentlichen oberhalb des Transportkanals 31. Bei einemHerunterschwenken der Faltklappe 50 kommt die Faltklappe, 50 mit ihrer Unterseite 50d zumindest abschnittsweise auf der Oberseite 40c des Magazin 40 und/oder auf der Oberseite 60c der Halteklappe 60 zum Liegen auf. An der Unterseite 50d der Faltklappe 50 ist die Faltrippe 51 positioniert. Diese greift durch die Öffnung 62 in der Halteklappe 60 hindurch in. das Magazin 40 ein und drückt die Linse 90 nach unten, in den Transportkanal 31 der Kartusche 80. Die Kartusche 80 ist in einem entsicherten Zustand dargestellt. Der Injektor 100 ist betriebsbereit. Durch ein Betätigen des Schiebers 20 kann die Linse 90 aus dem Transportkanal 31 heraus gedrückt werden. Der Scheitel der Faltrippe 50 liegt unterhalb der Oberseite des Transportkanals 31. Dadurch kann die Gefahr eines möglichen Verklemmens der Linse 90 im Transportkanal 31 vermieden werden.

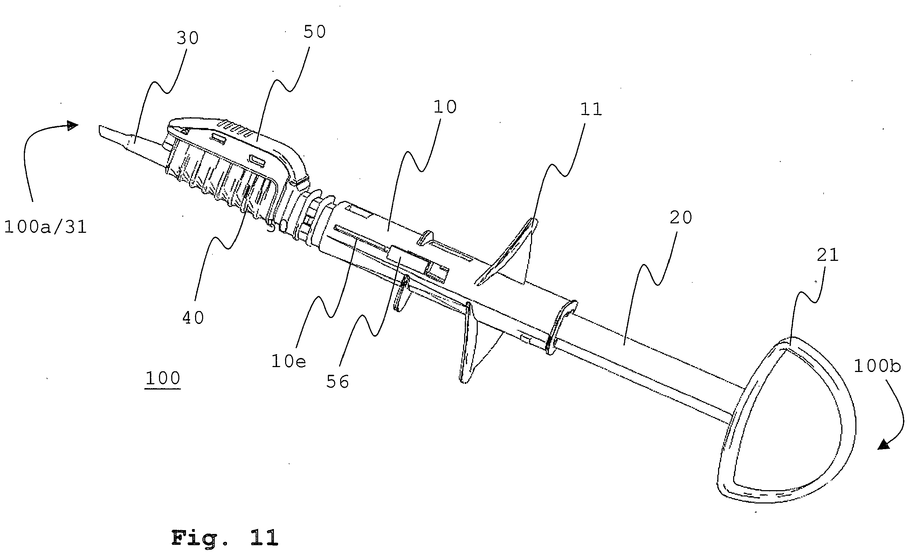

Weiterhin wird in den Figuren 11 bis 14 eine weitere Ausführungsform des Injektors 100 präsentiert. Dieser besitzt einen Faltkörper 51 mit einem Aufnahmebereich 54für die hintere Haptik 91 der Linse 90 und einen Anschlag 55 für die vordere Haptik 91 der Linse 90. Die hintere Haptik 91 der Linse 90 und der zugeordnete Aufnahmebereich 54 liegen in Richtung der Rückseite 100b des Injektors 100. Die vordere Haptik 91 und der Anschlag 55 liegen in

Richtung der Vorderseite 100a des Injektors 100. Die Figur 11 zeigt zunächst den Injektor 100 in einer Außenansicht. Die Veränderungen am Faltkörper 51 sind in dieser Darstellung des Injektors 100 nicht sichtbar (siehe hierzu die nachfolgenden Figuren) . Zu erkennen ist die Betätigungseinrichtung des Haptikschiebers 56. Der Injektorkörper 10 weist hierzu in einer Seite eine Öffnung 10e, hier ausgebildet als ein Schlitz, auf, durch welche sich die Betätigungseinrichtung des Haptikschiebers 56 erstreckt. Durch eine Bewegung des Haptikschiebers 56 in Richtung der Vorderseite 100a des Injektors 100 kann die hintere Haptik 91 in den Aufnahmebereich 54 des Faltkörpers 51 eingeschoben werden.

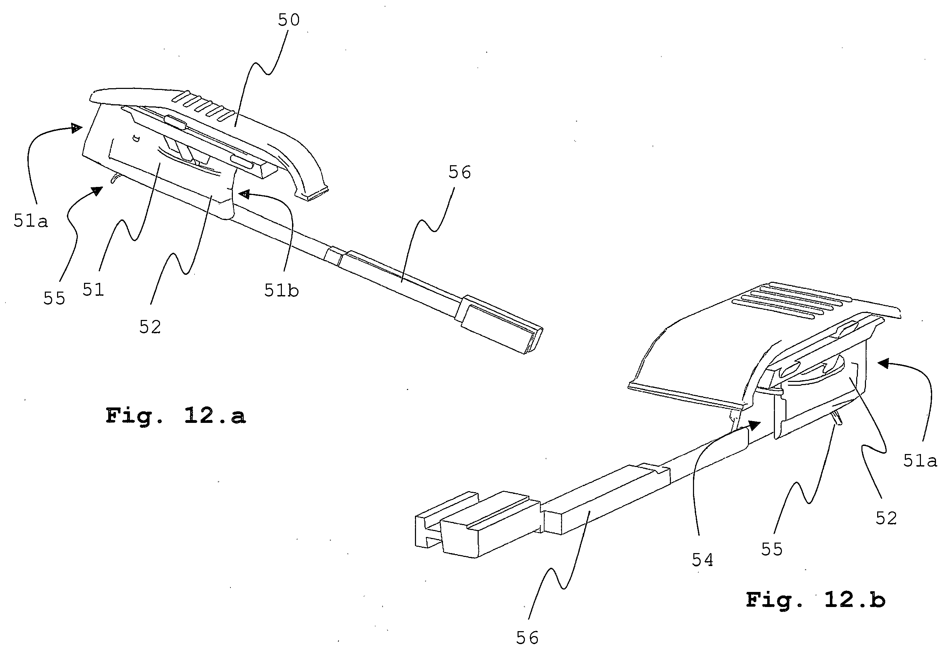

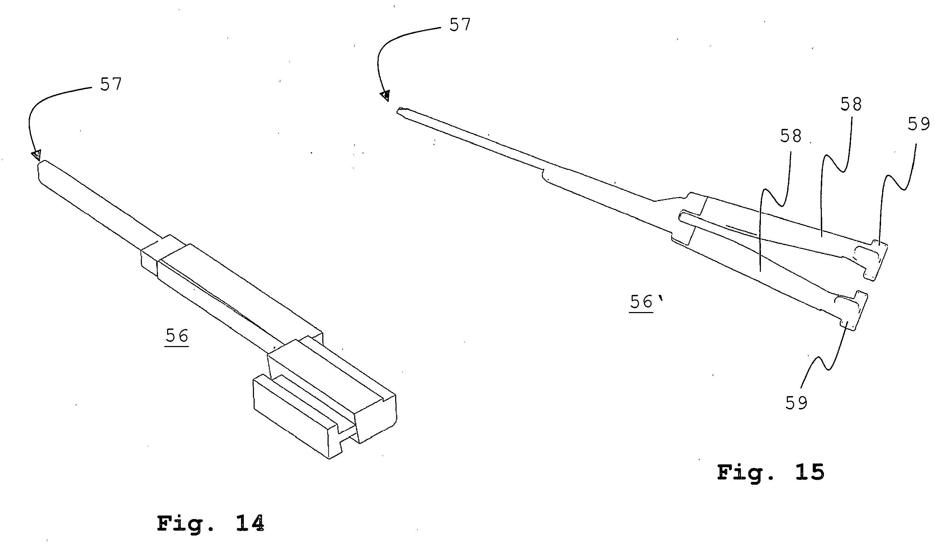

Zum besseren Verständnis zeigen die Figuren 12. a und 12. b den Faltkörper 51 und den Haptikschieber 56 alleine ohne die weiteren Injektorbauteile in zwei seitlichen Ansichten. Die Linse 90 mit ihren Haptiken 91 ist aus Gründen einer besseren Übersicht nicht eingezeichnet. Für den Aufbau des Haptikschiebers 56 sei auf die Ausführungen zur Figur 14 verwiesen.

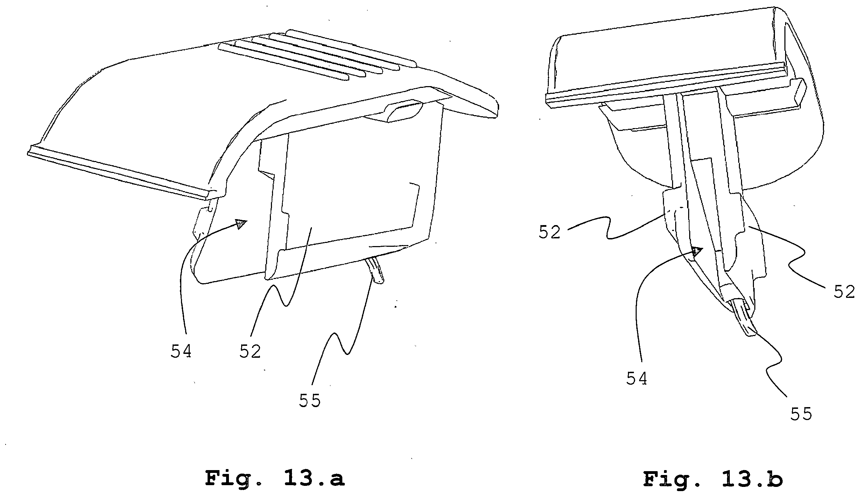

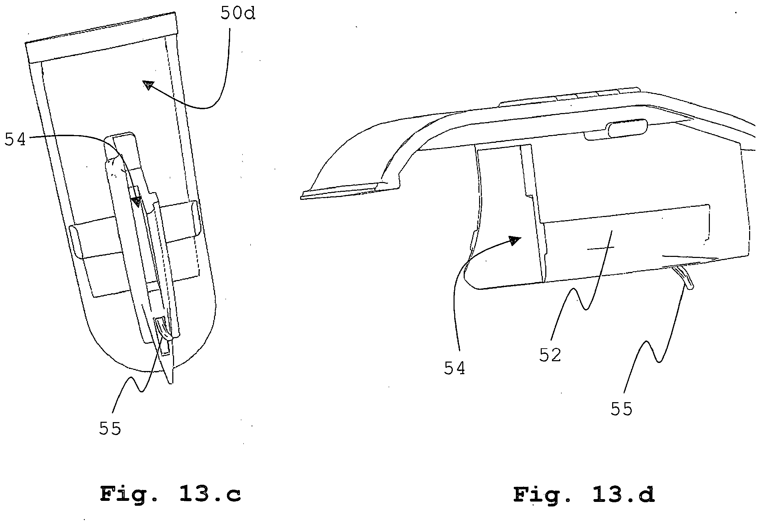

In Figur 12. b ist der Aufnahmebereich 54 für die hintere Haptik 91 der Linse 90 sichtbar. Der Anschlag 55 für die vordere Haptik 91 der Linse 90 ist sowohl in Figur 12. a als auch in 12. b sichtbar. Der "erweiterte" Faltkörper 51 ist in den Figuren 13. a bis 13. d aus verschiedenen Perspektiven dargestellt Der Aufnahmebereich 54 für die hintere Haptik 91 ist als eine Aussparung in dem Faltkörper 51 ausgeführt. Die Aussparung 54 erstreckt sich von der Rückseite 51b bis zur Unterseite 51d des Faltkörpers 51. Sie erstreckt sich zumindest abschnittsweise durch den Faltkörper 51.

Vorzugsweise erstreckt sie sich aber nicht vollständig • durch den Faltkörper 51, insbesondere von der Rückseite51b bis zur Vorderseite 51a. In einer Ausführungsform besitzt die Aussparung 54 ausgehend von der Rückseite 51b des Faltkörpers 51 in Richtung der Vorderseite 51a des Faltkörpers 51 eine abnehmende Tiefe. Sie mündet in der Unterseite 51d des Faltkörpers 51. Die dadurch im Inneren des Faltkörpers 51 gebildete Rampe kann zum Beispiel zumindest abschnittsweise linear oder gekrümmt sein.

Der Anschlag 55 für die vordere Haptik 91 ist als ein Vorsprung oder eine Art Finger ausgeführt. Der Vorsprung 55 ist an der Unterseite 51d des Faltkörpers 51 positioniert. Der Vorsprung 55 ist vorzugsweise flexibel, insbesondere elastisch. In der dargestellten Ausführungsform besitzt der Vorsprung 55 die Gestalt eines Bogens mit einer Krümmung in Richtung der Vorderseite 51a. Durch das Einfügen des Faltkörpers 51 wird die Linse 90 zum einen um den Faltkörper 51 gefaltet und zum anderen in den Transportkanal 32 überführt. Um ein sicheres Einbringen der Linse 90 in ein Auge zu unterstützen, ist es vorteilhaft, wenn sich die Haptiken 91 der Linse 90 in einer definierten Position befinden.

Das wird erreicht durch den Aufnahmebereich 54, welcher der hinteren Haptik 91 zugeordnet ist, und den Anschlag 55, der der vorderen Haptik 91 zugeordnet ist. Durch den Haptikschieber 56 wird die hintere Haptik 91 in den Aufnahmebereich 54 eingeführt. Dort befindet sich die hintere Haptik 91 in einer definierten Ausgangsposition. In einem nächsten Schritt wird der Schieber 20 zum Ausbringen der Linse 90 aus dem Injektor 100 betätigt. Der Schieber 20 schiebt die Linse 90 nach vorne. Die hintere Haptik 91 wird aus dem Aufnahmebereich 54 herausgezogen und fügt sich zum Beispiel in die gefaltete Linse 90 ein. Bei einem weiteren Vorschieben, welches zu einem Aufrollen der Linse 90 führt, wird die hintere Haptik 91 in die Linse 90 eingerollt. Sie besitzt somit beim Auswerfen der Linse 90 eine definierte Position . Beim Vorschub wird zusätzlich die vordere Haptik 91 auf den Anschlag 55 geführt. Durch ein weiteres .Vorschieben der Linse 90 legt oder drückt der Anschlag 55 die vordere Haptik 91 auf die Linse 90. Da der Anschlag 55 vorzugsweise flexibel ist, kann dieser beim weiteren Vorschub durch die Linse 90 zur Seite gedrückt werden. Der Anschlag 55 kann auch so oberhalb der Linse 90 angeordnet sein, dass lediglich die vordere Haptik 91 mit ihm einen Kontakt hat, die Optik der Linse 90 bzw. die Linse 90 selbst aber nicht. Der Haptikschieber 56 ist in dem Injektorkörper 10 oberhalb des Schiebers 20 für die Linse 90 angeordnet. Hierzu zeigt Figur 14 eine erste Ausgestaltung des Haptikschiebers 56. Er ist hier im Wesentlichen L-förmig aufgebaut. Der kurze Arm erstreckt sich durch die Außenseite des Injektorkörpers 10 und stellt eine Betätigungseinrichtung oder eine Art Handgriff für den Haptikschieber 56 bereit. An dem langen Arm kann der Haptikschieber 56 einen hier nicht gezeigten Fänger 57 für die hintere Haptik 91 besitzen. Der Fänger 57kann zum Beispiel durch eine, vorzugsweise zweizackige, Gabel bereitgestellt werden.

Die hintere Haptik 91 der Linse 90 kann über ein separates Betätigen des Haptikschieber 56 oder über ein gekoppeltes Betätigen von Haptikschieber 56 und Schieber 20 in den Aufnahmebereich 54 überführt werden. Die in Figur 14 gezeigte Ausführungsform des Schieber 56 ist insbesondere für das separate Betätigen geeignet. Sie kann aber auch für eine Kopplung mit dem Schieber 20 eingesetzt werden.

Wie es vorstehend bereits ausgeführt ist, kann die Bewegung des Haptikschiebers 54 aber auch mit der Bewegung des Schiebers 20 für die Linse 90 gekoppelt sein oder werden. Dabei können alleine über die Betätigung des Schiebers 20 zunächst die hintere Haptik 91 der Linse 91 in den Aufnahmebereich 54 überführt und dann die Linse 90 aus dem Injektor 100 ausgeschoben werden. Die Figur 15 zeigt eine Ausführungsform des Haptikschiebers 56', der insbesondere für eine Kopplung mit dem Schieber 20 für die Linse 90, welcher nachfolgend kurz Linsenschieber 20 genannt wird, geeignet ist. Diese Ausführungsform kann auch von dem Haptikschieber 56 separat betätigt werden. Der Haptikschieber 56' besitzt an seiner Rückseite zwei Biegearme 58. Er ist im Wesentlichen Y-förmig ausgeführt. Die folgenden Ausführungen beschreiben einen Biegearm 58, beziehen sich aber auf beide Biegearme 58. Das Ende des Biegearms 58 ist hier im Wesentlichen T-förmig. Es wird damit ein Vorsprung 59 zur Außenseite und ein Vorsprung zur Innenseite bereitgestellt.

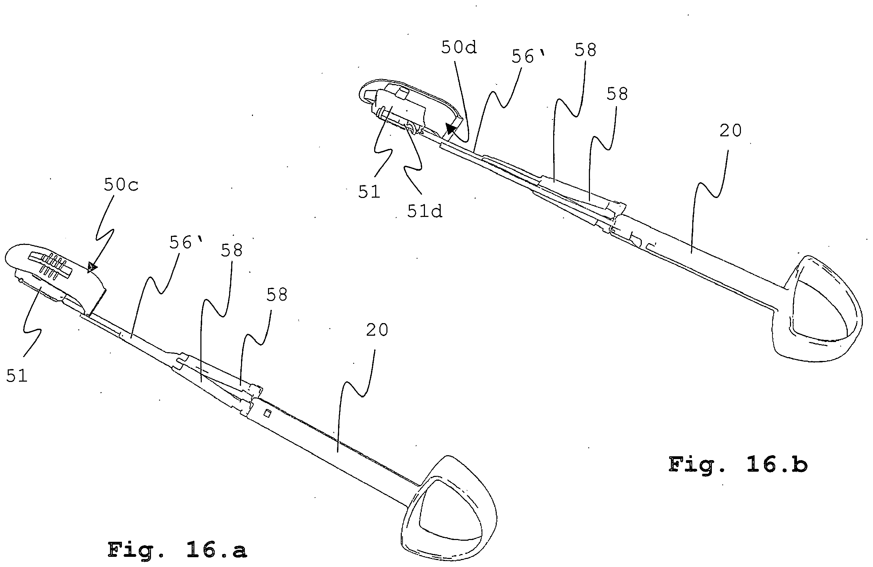

Die Figuren 16. a und 16. b zeigen den Haptikschieber 56' aus Figur 15 in Wirkverbindung mit dem Linsenschieber 20 und dem Faltkörper 51. Wenn der Linsenschieber 20 betätigt wird, trifft er zunächst auf den Haptikschieber 56', insbesondere die Biegearme 58. Der Linsenschieber 20 kann aber auch bereits in seiner Ausgangsstellung an dem Haptikschieber 56' anliegen. Der Linsenschieber 20 drückt die beiden Biegearme 58 gegen die Innenseite des hier nicht dargestellten Inj ektorkörpers 10. Im Detail drückt der Linsenschieber 20 die äußeren Vorsprünge 59 der Biegearme 58 gegen die Innenseite des Injektorkörpers 10. Durch das Bewegen des Linsenschiebers 20 wird der Haptikschieber 56' mit in Richtung der Vorderseite 100a des Injektors 100 bewegt. Der Haptikschieber 56' soll sich vorzugsweise nur soweit nach vorne bewegen, so dass die Haptik 91 in den Aufnahmebereich 54 überführt werden kann. Er soll dann von dem Linsenschieber 20 entkoppelt oder getrennt werden. Dies wird dadurch erreicht, dass in dem Inj ektorkörper 10 an einer der Position des Aufnahmebereichs 54 angepassten Position zwei Aussparungen lOf, insbesondere Öffnungen, eingebracht sind, in welche die Vorsprüngen 59 durch den Anspressdruck zum Eingriff kommen können. Durch dieses Eingreifen der Vorsprünge wird der Haptikschieber 56' von dem Linsenschieber 20 entkoppelt und gegebenenfalls arretiert. Der Linsenschieber 20 jedoch kann durch die Gabelung des Haptikschiebers 56' weiter nach vorne gleiten, die Linse 90 greifen und diese aus dem Injektor 100 ausstoßen.

Figur 17 zeigt den Injektor 100 mit dem Haptikschieber 56' aus Figur 15. Die Vorsprünge 59 des Haptikschiebers 56', die in den Löchern 10f des Injektorkörper 10 zum Eingriff gekommen sind und sich nun nach außen erstrecken, sind hier sichtbar. Dadurch wird der Haptikschieber 56' in seiner Position im Injektor 100 fixiert. Er bleibt sozusagenstehen. Er wird von der Bewegung des Linsenschiebers 20 entkoppelt.

Schließlich zeigt Figur 18 die erfindungsgemäße Ausfiihrungsform eines Faltkörpers 51 mit einen Aufnahmebereich 54 für die hintere Haptik 91 einer Linse 90. Zum einen mündet der Aufnahmebereich 54 in Richtung der Vorderseite 51a in eine Rille oder Rinne 54-1, welche in der Unterseite 51d des Faltkörpers 51 eingebracht ist. Dadurch wird das Einrollen der hinteren Haptik 91 durch die Linse 90 verbessert. Zum anderen ist der Faltkörper 51 hier zweiteilig aufgebaut. Es wird eine Art Sockel 54-2 bereitgestellt, in welchem das eigentliche Faltelement 54-3 mit dem Haptikaufnahmebereich 54 eingesteckt ist. Das Faltelement 54-3 ist von den in dem Faltkörper integrierten Rückhaltekanten 52 separiert. Die Rückhaltekanten 52 werden vom Sockel 54-2 bereitgestellt.

Dieser Aufbau erlaubt eine Erhöhung der Sicherheit. Die Trennung in zwei Teile erlaubt eine Erhöhung des Abstandes zwischen den Rückhaltekanten 52 und der Unterseite 51d der Faltrippe. 51. Dies kann ein Einklemmen der Linse 90 während des Faltprozesses im Wesentlichen ausschließen. Das Faltelement 54-3 ist in dem Sockel 54-2 in Richtung zur Oberseite50c verschiebbar angeordnet. Dies wird dadurch erzielt, dass die Oberseite des Faltelements 54-3 im Inneren des Sockels 54-2 nicht an diesem anliegt.- Es wird ein Zwischenraum bereitgestellt. In dem Zwischenraum kann zudem ein flexiber Körper, zum Beispiel ein Schaumstoff, eingebracht sein. Dadurch kann ein Druck auf das Faltelement 54-3 ausgeübt werden. Das Faltelement 54-3 kann die Linse 90 beim Auftreffen auf diese bis zum Boden des Transportkanals 31 transportieren, insbesondere unabhängig von der Dicke der Linse 90. Beim Auftreffen auf den Boden des Transportkanals 32 kann dann das Faltelement 54-3 nach oben weiter in den Sockel 54-2 eingedrückt werden. Durch einen Vorschub des Kolbens 20 kann das Faltelement 54-3 in eine gemeinsame Dachlinie mit dem Sockel 54-2 gedrängt werden. Dadurch entsteht ein im Wesentlichen gleichförmiger Kanal, der den Vorschub zum Beispiel mit einem Silikonpuffer erleichtert.

### Bezugszeichenliste:

- 10: Injektorkörper oder Injektorgehäuse oder Gehäuse oder Handstück
- 10a: Vorderseite des Injektorkörpers
- 10b: Rückseite des Injektorkörpers
- 10c: Oberseite des Injektorkörpers
- lOd: Unterseite des Injektorkörpers
- lOe: Schlitz im Injektorkörper
- lOf: Aussparung oder Öffnung im Injektorkörper
- 11: Griff am Injektorkörper
- 12: Aussparung im Injektorkörper
- 13: Führungsmittel oder Führungsschiene für den Schieber
- 14: Loch im Injektorkörper20 Schieber oder Kolben oder Linsenschieber 20a Vorderseite des Schiebers
- 20b: Rückseite des Schiebers
- 20-1: Erster Abschnitt des Schiebers
- 20-2: Zweiter vorderer Abschnitt des Schiebers
- 21: Griff oder Schiebergriff
- 22: Schieberstange oder Schieberarm
- 23: Führungsmittel für die Linse oder Gabel
- 24: Aufnahmebereich für die Linsenhaptik oder Aussparung
- 25: Vorsprung an dem Schieber oder Rastnase
- 30: Kanüle oder Einführrohr in das Auge oder Ausführkörper für die Linse
- 30a: Vorderseite der Kanüle
- 30b: Rückseite der Kanüle
- 30c: Oberseite der Kanüle
- 30d: Unterseite der Kanüle
- 31: Transportkanal oder Vorschubkanal
- 32: Seitliche Eingangsöffnung
- 33: Vorsprung an der Kanüle oder Rastnase
- 40: Magazin oder Behälter zum Lagern der Linse
- 40a: Vorderseite des Magazins
- 40b: Rückseite des Magazins
- 40c: Oberseite des Magazins
- 40d: Unterseite des Magazins 41 Hohlraum oder Innenraum des Magazins
- 42: Boden des Magazins
- 43: Wand des Magazins
- 44: Aufnahmebereich für eine Linse und eine Haptik oder Aussparung in der MagazinwandFührungsbereich und/oder Aufnahmebereich für eineHaptik einer Linse oder Aussparung oder Kanal in der Magazinwand
Aussparung oder Öffnung in der Magazinwand
Aussparung oder Öffnung in der Magazinwand Faltklappe oder Faltplattenträger oder Klappe
- a: Vorderseite der Faltklappe
- b: Rückseite der Faltklappe
- c: Oberseite der Faltklappe'
- d: Unterseite der Faltklappe Faltkörper oder Faltplatte oder Faltrippe
- a: Vorderseite des Faltkörpers
- b: Rückseite des Faltkörpers
- d: Unterseite der Faltplatte Rückhaltekante an der Faltplatte
- d: Unterseite der Rückhaltekante Vorsprung an der Faltklappe Aufnahmebereich oder Aussparung am Faltkörper
- -1: Rille im Faltkörper
- -2: Sockel des Faltkörpers
- -3: Faltelement des Faltkörpers Anschlag oder Vorsprung am Faltkörper Haptikschieber in L-Form 'Haptikschieber in Y-Form Fänger oder Greifer am Haptikschieber Biegearm des Haptikschiebers Vorsprung am Biegearm60 Halterung oder Halteklappe oder Klappe
- 60c: Oberseite der Halterung
- 60d: Unterseite der Halterung 61 Schenkel der Halterung
- 62: Öffnung oder Loch in der Halterung
- 63: Vorsprung an der Halteklappe
- 64: Rückhalteeinrichtung oder Mittel zum Arretieren oder Festsetzen der Linse oder Fixiereinrichtung
- 65: Verdrehsicherung oder Stift oder Vorsprung
- 66: Zwischenstück oder Träger oder Rippe
- 80: Kartusche oder Injektionskartusche 90 Linse oder Intraokularlinse
- 91: Haptik der Linse
- 100: Injektor oder Injektorsystem oder Applikator
- 100a: Vorderseite des Injektors
- 100b: Rückseite des Injektors

## Patentansprüche

1. Injektorsystem (100) zum Implantieren einer Linse (90) in ein Auge umfassend
- einen Injektorkörper (10) mit einer Vorderseite (10a) und einer Rückseite (10b),
- eine an der Vorderseite (10a) des Injektorkörpers (10) angeordnete Kanüle (30), die einen Transportkanal (31) für die zu implantierende Linse (90) bereitstellt, wobei dem Transportkanal (31) die Linse (90) über eine Eingangsöffnung (32) zuführbar ist,
- ein Magazin (40) mit einem Aufnahmebereich (44) für wenigstens die Linse (90), welche in dem Aufnahmebereich (44) mittels einer Halterung (60) festsetzbar ist, wobei das Magazin (40) so angeordnet ist, dass dem Transportkanal (31) die Linse (90) über die Eingangsöffnung (32) zuführbar ist,
- einen in das Magazin (40) und die Eingangsöffnung (32) einfügbaren Faltkörper (51) zum Einschieben der Linse (90) in den Transportkanal (32) derart, dass die Linse (90) zumindest abschnittsweise um den Faltkörper (51) faltbar ist, und
- einen Schieber (20), welcher in dem Injektorkörper (10) verschiebbar angeordnet ist und über die Vorderseite (10a) des Injektorkörpers (10) so in den Transportkanal (31) einschiebbar ist, dass die Linse (90) aus dem Transportkanal (31) ausgeschoben werden kann,
**dadurch gekennzeichnet, dass** der Faltkörper (51) zumindest zweiteilig aufgebaut ist und durch einen Sockel (54-2) und ein Faltelement (54-3) bereitgestellt wird, wobei das Faltelement (54-3) in dem Sockel (54-2) verschiebbar in Richtung zu einer Oberseite (50c) angeordnet und in dem Sockel (54-2) eingesteckt ist, wobei eine Oberseite des Faltelements (54-3) im Inneren des Sockels (54-2) nicht an diesem anliegt, so dass ein Zwischenraum erzeugt ist.

2. Injektorsystem (100) nach dem vorstehenden Anspruch, **dadurch gekennzeichnet, dass** das Magazin (40) zum Laden des Injektorsystems (100) mit der Linse (90) mit der Kanüle (30) so verbindbar ist, dass die Kanüle (30) zumindest abschnittsweise im Inneren (41) des Magazins (40) oder am Magazin (40) angeordnet ist und das Magazin (40) mit dem Injektorsystem (100) einrastend verbunden ist.

3. Injektorsystem (100) nach vorstehendem Anspruch, **dadurch gekennzeichnet, dass** die Halterung (60) als eine Klappe (60) ausgeführt ist, die schwenkbar an dem Magazin (40) angeordnet ist, wobei in einer geschlossenen Stellung der Klappe (60) die Linse (90) in dem Aufnahmebereich festsetzbar ist.

4. Injektorsystem (100) nach einem der vorstehenden Ansprüche, **dadurch gekennzeichnet, dass** das Magazin (40) zum Laden des Injektorsystems (100) mit der Linse (90) auf die Kanüle (30) so aufschiebbar ist, dass die Kanüle (30) zumindest abschnittsweise im Inneren (41) des Magazins (40) oder am Magazin (40) angeordnet ist.

5. Injektorsystem (100) nach einem der vorstehenden Ansprüche, **dadurch gekennzeichnet, dass** die Halterung (60) an der Seite (60d), welche der Linse (90) zugeordnet ist, eine Rückhalteeinrichtung (64) und/oder eine Verdrehsicherung (65) für die in dem Aufnahmebereich (44) zu lagernde Linse (90) aufweist.

6. Injektorsystem (100) nach vorstehendem Anspruch 4, **dadurch gekennzeichnet,**
**dass** die Rückhalteeinrichtung (64) als ein Vorsprung (64) ausgeführt ist, der sich zumindest abschnittsweise über den Umfang der in dem Aufnahmebereich (44) festzusetzenden Linse (90) erstreckt und eine zu dieser im Wesentlichen korrespondierende Krümmung aufweist und/oder
**dass** die Verdrehsicherung (65) als wenigstens ein Stift (65) ausgeführt ist, welcher zwischen der in dem Aufnahmebereich (44) angeordneten Linse (90) und einer Haptik (91) der Linse (90) eingreift und an der Rückhalteeinrichtung (64) angeordnet ist.

7. Injektorsystem (100) nach einem der vorstehenden Ansprüche, **dadurch gekennzeichnet,**
**dass** die Halterung (60) eine Öffnung (62) aufweist und der Faltkörper (51) über die Öffnung (62) in der Halterung (60) in das Magazin (40) und in die Eingangsöffnung (32) in dem Transportkanal (31) einfügbar ist und/oder
**dass** der Faltkörper (51) an einer Unterseite (50d) einer Klappe (50) angeordnet ist, welche schwenkbar an der Kanüle (30) befestigt ist, so dass die Linse (90) in einem heruntergeklappten Zustand der Klappe (50) in den Transportkanal (31) einschiebbar und zumindest abschnittsweise um die Faltplatte (51) faltbar ist.

8. Injektorsystem (100) nach einem der vorstehenden Ansprüche, **dadurch gekennzeichnet, dass** an dem Faltkörper (51) und/oder in dem Transportkanal (31) wenigstens zwei Rückhaltekanten (52) für die Linse (90) angeordnet sind, welche eine Anlagefläche (52d) wenigstens für eine Kante der Linse (90) beim Einschieben der Linse (90) in den Transportkanal (31) definieren.

9. Injektorsystem (100) nach einem der vorstehenden Ansprüche, **dadurch gekennzeichnet,**
**dass** der Faltkörper (51) einen Aufnahmebereich (54) für eine hintere Haptik (91) der Linse (90) aufweist, in welchen die hintere Haptik (91) der Linse (90) einführbar ist und/oder
**dass** der Aufnahmebereich (54) für die hintere Haptik (91) der Linse (90) durch eine Aussparung an einer Rückseite (51b) des Faltkörpers (51) bereitgestellt wird.

10. Injektorsystem (100) nach vorstehendem Anspruch 8, **gekennzeichnet**
**durch** einen Haptikschieber (56, 56') zum Einführen der hinteren Haptik (91) in den Aufnahmebereich (54) des Faltkörpers (51) und/oder
**dadurch**, dass der Haptikschieber (56, 56') mit dem Schieber (20) für die Linse (90) gekoppelt oder koppelbar ist, so dass die hintere Haptik (91) der Linse (90) durch den Haptikschieber (56, 56') mittels einer Bewegung des Schiebers (20) für die Linse (90) in den Aufnahmebereich (54) des Faltkörpers (51) einschiebbar ist.

11. Injektorsystem (100) nach vorstehendem Anspruch 9, **dadurch gekennzeichnet,**
**dass** der Haptikschieber (56') wenigstens einen Biegearm (58) umfasst, an welchem der Schieber (20) anliegt oder beim Ausschieben der Linse (90) zur Anlage kommt, so dass der wenigstens eine Biegearm (58) gegen eine Innenseite des Injektorkörpers (10) gedrückt wird und der Haptikschieber (56') durch den Schieber (20) mit in Richtung der Vorderseite (100a) des Injektors (100) bewegbar ist und/oder
**dass** der wenigstens eine Biegearm (58) wenigstens einen Vorsprung (59) aufweist, über welchen der Biegearm (58) gegen die Innenseite des Injektorkörpers (10) gedrückt wird.

12. Injektorsystem (100) nach vorstehendem Anspruch 10, **dadurch gekennzeichnet,**
**dass** der wenigstens eine Biegearm (58) nach einer Verschiebung des Haptikschiebers (56') in Richtung der Vorderseite (100a) des Injektors (100) in einer Aussparung (10f) der Innenseite des Injektorkörpers (10) zum Eingriff kommt, so dass der Haptikschieber (56') von dem Schieber (20) für die Linse (90) entkoppelbar ist und/oder
**dass** der Haptikschieber (56, 56') an einer Vorderseite einen Fänger (57) für die hintere Haptik (91) der Linse (90) besitzt.

13. Injektorsystem (100) nach einem der vorstehenden Ansprüche, **dadurch gekennzeichnet,**
**dass** der Faltkörper (51) einen Anschlag (55) für eine vordere Haptik (91) der Linse (90) aufweist, so dass die vordere Haptik (91) beim Ausschieben der Linse (90) durch den Schieber (20) mittels des Anschlags (55) auf der Linse (90) zur Auflage kommt und/oder
**dass** der Anschlag (55) für die vordere Haptik (91) der Linse (90) als ein flexibler Vorsprung an einer Unterseite (51d) des Faltkörpers (51) bereitgestellt wird.

## Claims

1. An injector system (100) for implanting a lens (90) into an eye, comprising:
- an injector body (10) having a front end (10a) and a rear end (10b);
- a cannula arranged at the front end (10a) of the injector body (10), which provides a transport channel (31) for a lens (90) to be implanted, wherein a lens (90) can be fed into the transport channel (31) via an inlet opening (32);
- a magazine (40) having a receptacle area (44) for at least one lens (90) which can be secured in the receptacle area (44) by means of a retainer (60), wherein the magazine (40) is arranged in a manner so that a lens (90) can be fed into the transport channel (31) via the inlet opening (32);
- a folding body (51) which is insertable into the magazine (40) and into the inlet opening (32), for pushing the lens (90) into the transport channel (32) in such a manner that the lens (90) is at least partially foldable around the folding body (51); and
- a slider (20) which is slideably arranged in the injector body (10), preferably axially, and can be pushed into the transport channel (31) via the front end (10a) of the injector body (10) in such a way that the lens can be ejected from the transport channel ( 31),
**characterized in that** the folding body (51) comprises at least two parts and is provided by a base (54 - 2) and by a folding member (54 - 3), wherein the folding member (54 - 3) is arranged to be displaceable in direction of an upper surface (50c) and is inserted into the base (54 - 2), wherein an upper surface of the folding member (54-3) inside the interior of the base ( 54 - 2) does not touch the base, thereby forming a gap.

2. The injector system (100) as claimed in the preceding claim, wherein for loading the injector system (100) with a lens (90), the magazine (40) is connectable to the cannula (30), and is preferably push-fittable to the cannula (30) in a manner so that the cannula (30) is at least partially arranged in the interior (41) of the magazine (40) or on the magazine (40).

3. The injector system (100) as claimed the preceding claim, wherein the retainer (60) is configured as a flap ( 60) which is preferably pivotally mounted to the magazine ( 40), wherein in a closed position of the flap (60) a lens ( 90) can be secured in the receptacle area.

4. The injector system (100) as claimed in the preceding claim, wherein the magazine (40) for loading the injector system (100) can be push-fitted over the cannula (30) of the injector system (100) in a manner, so that the cannula ( 30) is at least partially arranged in the interior (41) of the magazine (40) or on the magazine (40).

5. The injector system (100) as claimed in claim 1, wherein at the side (60 d) associated with the lens (90), the retainer (60) has a retaining device (64) and/or an anti-rotation protection (65) for a lens (90) to be stored in the receptacle area (44).

6. An injector system (100) as claimed in claim 4, wherein the retaining device (64) is formed as a projection (64) at least partially extending over the periphery of a lens (90 ) to be secured in the receptacle area (44) and having a curvature which substantially corresponds to that of the lens; and/or wherein
the anti-rotation protection (65) is configured as at least one pin (65) which engages between a lens (90) arranged in the receptacle area (44) and a haptic (91) of said lens ( 90), and which is preferably arranged at the retaining device (64).

7. The injector system (100) as claimed in any of the preceding claims, wherein
the retainer (60) has an opening (62), and the folding body (51) is insertable into the magazine (40) and into the inlet opening (32) of the transport channel (31) through said opening (62) in the retainer (60) and/or wherein the folding body (51) is arranged at a lower side ( 50d) of a flap (50) which is preferably pivotally mounted to the cannula (30) in a manner so that in a hinged-down condition of the flap (50) a lens (90) is disposed in the transport channel (31) and is folded, at least partially, around the folding body (51).

8. The injector system (100) as claimed in any of the preceding claims, wherein at least two retaining ledges (52) for a lens (90) are provided at the folding body (51) and/or in the transport channel (31), which define an engagement surface (52d) at least for one edge of a lens ( 90) upon insertion of a lens (90) into the transport channel (31).

9. The injector system (100) as claimed in any of the preceding claims, wherein the folding body (51) has a receptacle area (54) for a trailing haptic (91) of the lens (90) into which the trailing haptic (91) of the lens (90) can be introduced
and/or wherein the receptacle area (54) for the trailing haptic (91) of the lens (90) is provided by a recess at a rear end (51b) of the folding body (51).

10. The injector system (100) as claimed in claim 8, wherein the injector system (100) comprises a haptic slider (56, 56') for inserting the trailing haptic (91) into the receptacle area (54) of the folding body (51).
and/or wherein the haptic slider (56, 56') is or can be coupled with the slider (20) for the lens (90) in a manner so that by moving the slider (20) for the lens (90), the trailing haptic (91) of the lens (90) can be pushed into the receptacle area (54) of the folding body (51) by means of the haptic slider (56, 56').

11. The injector system (100) as claimed in claim 9, wherein the haptic slider (56') comprises at least one bending arm (58) that is engaged by the slider (20) or that comes in engagement with the slider (20) when pushing out the lens (90), so that the at least one bending arm (58 ) is biased against an inner surface of the injector body (10 ) and the haptic slider (56') is movable together with the slider (20) towards the front end (100a) of the injector (100), and/or wherein the at least one bending arm (58) has at least one projection (59) through which the bending arm (58) is biased against the inner surface of the injector body (10).

12. The injector system according to claim 10,
wherein when displacing the haptic slider (56') towards the front end (100a) of the injector (100), the at least one bending arm (58) engages into a recess (10f) in the inner surface of the injector body (10), so that the haptic slider (56') can be decoupled from the slider (20) for the lens ( 90) and/or wherein the haptic slider (56, 56'), at a front end thereof, has a claw (57) for the trailing haptic (91) of the lens (90).

13. The injector system (100) as claimed in any of the preceding claims, wherein the folding body (51) has a stop ( 55) for a leading haptic (91) of the lens (90), so that when pushing out the lens (90) by means of the slider (20) the leading haptic (91) comes to rest on the lens (90), by means of the stop (55)and/or wherein the stop (55) for the leading haptic (91) of the lens (90) is provided as a preferably flexible lug at a lower end (51d) of the folding body (51).

## Revendications

1. Système d'injection (100) destiné à implanter une lentille (90) dans un œil, comprenant
- un corps d'injecteur (10) avec une partie avant (10a) et une partie arrière (10b),
- une canule (30) disposée sur la partie avant (10a) du corps d'injecteur (10), laquelle met à disposition un canal de transport (31) pour la lentille (90) à implanter, la lentille (90) pouvant être amenée au canal de transport (31) par un orifice d'entrée (32),
- un chargeur (40) avec une zone de réception (44) pour au moins la lentille (90) pouvant être fixée dans la zone de réception (44) au moyen d'un support (60), le chargeur (40) étant disposé de telle manière à ce que la lentille (90) puisse être amenée au canal de transport (31) par l'orifice d'entrée (32),
- un corps de pliage (51) pouvant être inséré dans le chargeur (40) et l'orifice d'entrée (32), pour l'insertion de la lentille (90) dans le canal de transport (31), de sorte que la lentille (90) puisse être pliée au moins en partie autour du corps de pliage (51),
- un coulisseau (20) qui est disposé de façon à pouvoir être déplacé dans le corps d'injecteur (10) et qui peut être inséré dans le canal de transport (31) par la partie avant (10a) du corps d'injecteur (10) de façon à ce que la lentille (90) puisse être extraite du canal de transport (31),
**caractérisé en ce que** le corps de pliage (51) est conçu au moins en deux parties et est mis à disposition par une base (54-2) et un élément de pliage (54-3), l'élément de pliage (54-3) étant disposé dans la base (54-2) de façon à pouvoir être déplacé vers une partie supérieure (50c) et étant inséré dans la base (54-2), une partie supérieure de l'élément de pliage (54-3) à l'intérieur de la base (54-2) n'étant pas appliqué sur cette dernière de façon à créer un interstice.

2. Système d'injection (100) selon la revendication précédente, **caractérisé en ce que** le chargeur (40) pour le chargement du système d'injection (100) avec la lentille (90) peut être relié à la canule (30) de façon à ce que la canule (30) soit disposée au moins en partie à l'intérieur (41) du chargeur (40) ou dans le chargeur (40) et à ce que le chargeur (40) soit relié par encliquetage au système d'injection (100).

3. Système d'injection (100) selon la revendication précédente, **caractérisé en ce que** le support (60) est conçu comme un clapet (60) pivotant sur le chargeur (40), la lentille (90) pouvant être fixée dans la zone de réception, le clapet (60) étant en position fermée.

4. Système d'injection (100) selon l'une des revendications précédentes, **caractérisé en ce que** le chargeur (40) pour le chargement du système d'injection (100) avec la lentille (90) peut être glissé sur la canule (30) de façon à ce que la canule (30) soit disposée au moins en partie à l'intérieur (41) du chargeur (40) ou dans le chargeur (40).

5. Système d'injection (100) selon l'une des revendications précédentes, **caractérisé en ce que** le support (60) présente, sur la partie (60d) qui est affectée à la lentille (90), un dispositif de retenue (64) et/ou une sécurité anti-rotation (65) pour la lentille (90) à déposer dans la zone de réception (44).

6. Système d'injection (100) selon la revendication 4 précédente, **caractérisé en ce que** le dispositif de retenue (64) est conçu comme une saillie (64) qui s'étend au moins en partie au-delà du pourtour de la lentille (90) à fixer dans la zone de réception (44) et présente une courbure correspondant sensiblement à cette dernière et/ou
**en ce que** la sécurité anti-rotation (65) est conçue au moins comme une tige (65) qui est en prise entre la lentille (90) disposée dans la zone de réception (44) et une prise (91) de la lentille (90) et qui est disposée sur le dispositif de retenue (64) .

7. Système d'injection (100) selon l'une des revendications précédentes, **caractérisé en ce que**
le support (60) présente un orifice (62) et le corps de pliage (51) peut être inséré, par l'orifice (62) dans le support (60), dans le chargeur (40) et dans l'orifice d'entrée (32) dans le canal de transport (31) et/ou
**en ce que** le corps de pliage (51) est disposé sur une partie inférieure (50d) d'un clapet (50) qui est fixé sur la canule (30) de façon à pouvoir pivoter, de telle manière à ce que la lentille (90) puisse être insérée dans le canal de transport (31), le clapet (50) étant rabattu, et puisse être pliée autour de la plaque de pliage (51).

8. Système d'injection (100) selon l'une des revendications précédentes, **caractérisé en ce que**, sur le corps de pliage (51) et/ou dans le canal de transport (31), au moins deux bords de retenue (52) sont disposés pour la lentille (90), lesquels définissent une surface de contact (52b) au moins pour un bord de la lentille (90) lors de l'insertion de la lentille (90) dans le canal de transport (31).

9. Système d'injection (100) selon l'une des revendications précédentes, **caractérisé en ce que** le corps de pliage (51) présente une zone de réception (54) pour une prise arrière (91) de la lentille (90), dans laquelle la prise arrière (91) de la lentille (90) peut être introduite et/ou
**en ce que** la zone de réception (54) pour la prise arrière (91) de la lentille (90) est mise à disposition par un évidement sur une face arrière (51b) du corps de pliage (51).

10. Système d'injection (100) selon la revendication 8 précédente, **caractérisé**
**par** un coulisseau de prise (56, 56') pour l'introduction de la prise arrière (91) dans la zone de réception (54) du corps de pliage (51) et/ou
en ce que le coulisseau de prise (56, 56') est couplé ou peut être couplé avec le coulisseau (20) pour la lentille (90) de telle sorte que la prise arrière (91) de la lentille (90) puisse être insérée dans la zone de réception (54) du corps de pliage (51) au moyen d'un mouvement du coulisseau (20) pour la lentille (90) par le coulisseau de prise (56, 56').

11. Système d'injection (100) selon la revendication 9 précédente, **caractérisé en ce que**
le coulisseau de prise (56') comprend au moins un bras flexible (58) sur lequel le coulisseau (20) est appliqué ou vient en prise lors de l'extraction de la lentille (90) de sorte qu'au moins un bras flexible (58) soit pressé contre une face intérieure du corps d'injecteur (10) et que le coulisseau de prise (56') puisse être déplacé vers la partie avant (100a) de l'injecteur (100) par le coulisseau (20) et/ou
**en ce qu'**au moins un bras flexible (58) présente au moins une saillie (59) au-dessus de laquelle le bras flexible (58) est pressé contre la face intérieure du corps d'injecteur (10).

12. Système d'injection (100) selon la revendication 10 précédente, **caractérisé en ce que**
au moins un bras flexible (58) est en prise après un déplacement du coulisseau de prise (56') vers la partie avant (100a) de l'injecteur (100) dans un évidement (10f) de la face intérieure du corps d'injecteur (10) de sorte que le coulisseau de prise (56') puisse être découplé du coulisseau (20) pour la lentille (90) et/ou
**en ce que** le coulisseau de prise (56, 56') possède, sur une partie avant, un récepteur (57) pour la prise arrière (91) de la lentille (90).

13. Système d'injection (100) selon l'une des revendications précédentes, **caractérisé en ce que**
le corps de pliage (51) présente une butée (55) pour une prise avant (91) de la lentille (90) de façon à ce que la prise avant (91) entre en contact sur la lentille (90) lors de l'extraction de la lentille (90) par le coulisseau (20) au moyen de la butée (55) et/ou
**en ce que** la butée (55) pour la prise avant (91) de la lentille (90) est mise à disposition comme une saillie souple sur une partie inférieure (51d) du corps de pliage (51).
